(19) Europäisches Patentamt
European Patent Office
Office européen des brevets

(11) **EP 3 774 999 B1**

(12) **EUROPÄISCHE PATENTSCHRIFT**

(45) Veröffentlichungstag und Bekanntmachung des Hinweises auf die Patenterteilung:
**03.08.2022 Patentblatt 2022/31**

(21) Anmeldenummer: **19713494.3**

(22) Anmeldetag: **01.04.2019**

(51) Internationale Patentklassifikation (IPC):
*A61L 15/18* (2006.01)    *A61L 15/54* (2006.01)
*A61L 15/60* (2006.01)    *C08J 3/075* (2006.01)
*C08J 3/24* (2006.01)    *C08K 3/22* (2006.01)

(52) Gemeinsame Patentklassifikation (CPC):
(C-Sets verfügbar)
**C08J 3/075; A61L 15/18; A61L 15/54; A61L 15/60; C08J 3/243; C08J 3/245;** C08K 2003/2227  (Forts.)

(86) Internationale Anmeldenummer:
**PCT/EP2019/058165**

(87) Internationale Veröffentlichungsnummer:
**WO 2019/197194 (17.10.2019 Gazette 2019/42)**

(54) **PERMEABLER SUPERABSORBER UND VERFAHREN ZU SEINER HERSTELLUNG**

PERMEABLE SUPERABSORBER AND METHOD FOR THE PRODUCTION THEREOF

SUPERABSORBANT PERMÉABLE ET PROCÉDÉ POUR SA FABRICATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.04.2018 EP 18166564**

(43) Veröffentlichungstag der Anmeldung:
**17.02.2021 Patentblatt 2021/07**

(73) Patentinhaber: **BASF SE**
**67056 Ludwigshafen am Rhein (DE)**

(72) Erfinder:
• **BAUDUIN, Christophe**
  **67056 Ludwigshafen (DE)**
• **DANIEL, Thomas**
  **67056 Ludwigshafen (DE)**

(74) Vertreter: **BASF IP Association**
**BASF SE**
**GBI-C006**
**67056 Ludwigshafen (DE)**

(56) Entgegenhaltungen:
**EP-A1- 1 209 186       EP-A1- 3 112 383**
**EP-A2- 0 233 067       WO-A1-99/55767**
**WO-A1-2010/108875      WO-A1-2019/091848**
**US-A1- 2016 235 882**

(52) Gemeinsame Patentklassifikation (CPC): (Forts.)

C-Sets
**C08K 3/22, C08L 33/02;**
**C08K 3/22, C08L 101/14**

**Beschreibung**

Die vorliegende Erfindung betrifft einen permeablen Superabsorbed, ein Verfahren zu seiner Herstellung sowie Verwendung und ihn enthaltende Hygienartikel.

Superabsorber sind bekannt. Für derartige Materialien sind auch Bezeichnungen wie "hochquellfähiges Polymer" "Hydrogel" (oft auch für die trockene Form verwendet), "Hydrogel bildendes Polymer", "Wasser absorbierendes Polymer", "absorbierendes gelbildendes Material", "quellfähiges Harz", "wasserabsorbierendes Harz" oder ähnliche gebräuchlich. Es handelt sich dabei um vernetzte hydrophile Polymere, insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboximethylcellulose, teilweise vernetztes Polyalkylenoxid oder in wässrigen Flüssigkeiten quellbare Naturprodukte, wie beispielsweise Guarderivate, wobei wasserabsorbierende Polymere auf Basis teilneutralisierter Acrylsäure am weitesten verbreitet sind. Die wesentlichen Eigenschaften von Superabsorbern sind ihre Fähigkeiten, ein Vielfaches ihres Eigengewichts an wässrigen Flüssigkeiten zu absorbieren und die Flüssigkeit auch unter gewissem Druck nicht wieder abzugeben. Der Superabsorber, der in Form eines trockenen Pulvers eingesetzt wird, wandelt sich bei Flüssigkeitsaufnahme in ein Gel, bei der üblichen Wasseraufnahme entsprechend in ein Hydrogel um. Die Vernetzung ist für synthetische Superabsorber wesentlich und ein wichtiger Unterschied zu üblichen reinen Verdickern, da sie zur Unlöslichkeit der Polymeren in Wasser führt. Lösliche Substanzen wären als Superabsorber nicht brauchbar. Das mit weitem Abstand wichtigste Einsatzgebiet von Superabsorbern ist das Absorbieren von Körperflüssigkeiten. Superabsorber werden beispielsweise in Windeln für Kleinkinder, Inkontinenzprodukten für Erwachsene oder Damenhygieneprodukten verwendet. Andere Anwendungsgebiete sind beispielsweise die als Wasser zurückhaltende Mittel im landwirtschaftlichen Gartenbau, als Wasserspeicher zum Schutz vor Feuer, zur Flüssigkeitsabsorption in Lebensmittelverpackungen oder ganz allgemein zur Absorption von Feuchtigkeit.

Superabsorber können ein Mehrfaches ihres Eigengewichts an Wasser absorbieren und unter gewissem Druck zurückhalten. Im Allgemeinen weist ein derartiger Superabsorber eine CRC ("Centrifuge Retention Capacity", Messmethode siehe unten) von mindestens 5 g/g, vorzugsweise mindestens 10 g/g und in besonders bevorzugter Form mindestens 15 g/g auf. Ein "Superabsorber" kann auch ein Gemisch stofflich verschiedener einzelner Superabsorber sein oder ein Gemisch von Komponenten, die erst im Zusammenwirken superabsorbierende Eigenschaften zeigen, es kommt hier weniger auf die stoffliche Zusammensetzung an als auf die superabsorbierenden Eigenschaften.

Wichtig für einen Superabsorber ist nicht nur seine Absorptionskapazität, sondern auch die Fähigkeit, Flüssigkeit unter Druck zurückzuhalten (Retention, meist als "Absorption unter Druck", "Absorption under Load" ("AUL") oder "Absorption against Pressure" ("AAP") ausgedrückt, Messmethode siehe unten) sowie die Permeabilität, also die Fähigkeit zur Flüssigkeitsweiterleitung im gequollenen Zustand (meist als "Saline Flow Conductivity" ("SFC") oder als "Gel Bed Permeability" ("GBP") ausgedrückt, Messmethoden siehe unten (wobei Veränderungen am Superabsorber seine SFC und GBP-Werte nicht unbedingt beide oder beide gleichermaßen verändern)). Gequollenes Gel kann die Flüssigkeitsweiterleitung zu noch nicht gequollenem Superabsorber behindern bis verhindern ("gel blocking"). Gute Weiterleitungseigenschaften für Flüssigkeiten besitzen beispielsweise Hydrogele, die im gequollenen Zustand eine hohe Gelfestigkeit aufweisen. Gele mit nur geringer Gelfestigkeit sind unter einem angewendeten Druck (Körperdruck) deformierbar, verstopfen Poren in dem Superabsorber / Cellulosefaser-Saugkörper und verhindern dadurch die Flüssigkeitsweiterleitung zu noch nicht oder nicht vollständig gequollenem Superabsorber und die Flüssigkeitsaufnahme durch diesen noch nicht oder nicht vollständig gequollenen Superabsorber. Eine erhöhte Gelfestigkeit wird in aller Regel durch einen höheren Vernetzungsgrad erreicht, wodurch allerdings die Absorptionskapazität des Produktes verringert wird. Eine elegante Methode zur Erhöhung der Gelfestigkeit stellt die Erhöhung des Vernetzungsgrads an der Oberfläche der Superabsorberpartikel gegenüber dem Inneren der Partikel dar. Dazu werden meist in einem Oberflächennachvernetzungsschritt getrocknete Superabsorberpartikel mit durchschnittlicher Vernetzungsdichte einer zusätzlichen Vernetzung in einer dünnen Oberflächenschicht ihrer Partikel unterworfen. Durch die Oberflächennachvernetzung steigt die Vernetzungsdichte in der Schale der Superabsorberpartikel, wodurch die Absorption unter Druckbelastung auf ein höheres Niveau gehoben wird. Während die Absorptionskapazität in der Oberflächenschicht der Superabsorberpartikel sinkt, weist ihr Kern durch das Vorhandensein beweglicher Polymerketten eine im Vergleich zur Schale verbesserte Absorptionskapazität auf, so dass durch den Schalenaufbau eine verbesserte Permeabilität gewährleistet wird, ohne dass gel blocking auftritt. Es ist ebenfalls bekannt, insgesamt höher vernetzte Superabsorber zu erzeugen und den Vernetzungsgrad im Inneren der Partikel gegenüber einer äußeren Schale der Partikel nachträglich zu verringern.

Auch Verfahren zur Herstellung von Superabsorbern sind bekannt. Superabsorber auf Basis von Acrylsäure, die auf dem Markt am gängigsten sind, werden durch radikalische Polymerisation von Acrylsäure in Gegenwart eines Vernetzers (dem "Innenvernetzer") hergestellt, wobei die Acrylsäure vor, nach oder teils vor, teils nach der Polymerisation zu einem gewissen Grad neutralisiert wird, üblicherweise durch Zugabe von Alkali, meist einer wässrigen Natriumhydroxidlösung. Das so gewonnene Polymergel wird zerkleinert (je nach verwendetem Polymerisationsreaktor kann dies gleichzeitig mit der Polymerisation erfolgen) und getrocknet. Das so gewonnene trockene Pulver (das "Grundpolymer" oder "Basispolymer") wird üblicherweise an der Oberfläche der Partikel nachvernetzt, indem es mit weiteren Vernetzern wie etwa organischen Vernetzern oder mehrwertigen Kationen, beispielsweise Aluminium (meist als Aluminiumsulfat eingesetzt) oder beidem umgesetzt wird, um eine gegenüber dem Partikelinneren stärker vernetzte Oberflächenschicht zu erzeugen.

Fredric L. Buchholz und Andrew T. Graham (Hrsg.) geben in: "Modern Superabsorbent Polymer Technology", J. Wiley & Sons, New York, U.S.A. / Wiley-VCH, Weinheim, Germany, 1997, ISBN 0-471-19411-5, einen zusammenfassenden Überblick über Superabsorber, ihre Eigenschaften und Verfahren zur Herstellung von Superabsorbern.

Superabsorber mit Aluminiumverbindungen zu behandeln ist bekannt. Beispielsweise werden Superabsorber mit anorganischen Feinteilchen bepudert, um die Verbackungsneigung herabzusetzen und die Fließfähigkeit des Pulvers oder auch seine Permeabilität zu erhöhen. Meist wird dafür gefälltes Siliziumdioxid verwendet, aber etwa aus US 7 795 345 B2, US 3 932 322 oder WO 2013/076031 A1 ist auch der Zusatz pyrogener Silicium- oder Aluminiumoxide zu Superabsorbern bekannt. Nach der Lehre von WO 01/68 156 A1 werden Superabsorbern Alumosilikate zugesetzt, beispielsweise Zeolithe, um die Permeabiltät zu steigern und unangenehme Gerüche zu binden. Die WO 2007/74 108 A1 lehrt, Superabsorber mit nichtreaktiven und nicht-filmbildenden Verbindungen zu überziehen, beispielsweise mit wasserunlöslichen Salzen. Hydratisiertes Aluminiumoxid wird in einer Reihe solcher Salze genannt. Die WO 2007/121 941 A2 offenbart ähnliche, mit anorganischen Pulvern überzogene Superabsorber, wobei die Pulver noch mit einem Binder versehen sein können. Aluminiumhydroxid wird in einer Reihe von anorganischen Pulvern genannt.

Es ist auch bekannt, wenn es auch nie bis höchstens selten in der Praxis gemacht wird, mehrwertige Kationen wie Aluminium als Innenvernetzer von Superabsorbern zu verwenden. Der Zusatz mehrwertiger Kationen zu Superabsorbern im Zuge der Oberflächennachvernetzung mit kovalente Bindungen zwischen den Polymerketten ausbildenden Oberflächennachvernetzungsmitteln ist dagegen üblich.

WO 99/55 767 A1 offenbart Superabsorber, denen vor, während oder nach der Polymerisation Aluminate der Formel $M_n[H_{2n+2}AlnO_{3n+1}]$ mit M = K oder Na und n = einer ganzen Zahl von 1 bis 10 zugegeben werden. WO 98/48 857 A1 beschreibt Superabsorber, die mit Al-, Fe-, Zr-, Mg- oder Zn-Kationen vernetzt und dann mit einer Flüssigkeit wie Wasser, Mineralöl oder Polyolen versetzt werden. WO 01/74 913 A1 betrifft die Regenerierung von Superabsorbern, spezifisch die Erhöhung einer durch Abrieb verringerten Permeabilität, durch Zugabe einer Lösung eines wenigstens dreiwertigen Kations, typischerweise einer wässrigen Aluminiumsulfatlösung.

US 6 620 889 B1 offenbart Superabsorber, die mit einer Kombination aus einem Polyol und einem Salz eines mehrwertigen Metalls in wässriger Lösung oberflächennachvernetzt werden. Das Anion des Salzes kann Chlorid, Bromid, Sulfat, Carbonat, Nitrate, Phosphat, Acetat oder Laktat sein. Die Verwendung von Aluminiumsulfat ist bevorzugt.

Nach der Lehre von WO 2006/111 402 A2 wird ein Grundpolymer mit einem Permeabilitätsverbesserer behandelt, der aus Silicium-Sauerstoff-Verbindungen, Salzen mehrwertiger, insbesondere dreiwertiger Kationen oder Mischungen davon gewählt wird. Das Salz eines dreiwertigen Kations ist vorzugsweise ein Aluminiumsalz, das aus einer Reihe von Salzen gewählt wird, die Aluminium-Laktat, -Oxalat, -Citrat, -Glyoxylat, -Succinat, -Tartrat und andere organische und anorganische Aluminiumsalze einschließt. WO 2005/108 472 A1 offenbart ein Verfahren, das die Behandlung eines Grundpolymers mit einem wasserlöslichen Salz eines mehrwertigen Metalls und einer organischen Säure oder ihrem Salz umfasst. Das Salz eines mehrwertigen Metalls ist vorzugsweise Aluminiumsulfat. Die organische Säure oder ihr Salz wird aus einer Reihe von Säuren gewählt, die Citronensäure, Glyoxylsäure, Glutarsäure, Bernsteinsäure, Weinsäure, Milchsäure sowie die Alkali- oder Ammoniumsalze dieser Säuren einschließt.

WO 2004/113 452 A1 beschreibt Superabsorber, die mit konzentrierten Lösungen von mehrwertigen Metallsalzen, insbesondere Natriumaluminiumat behandelt werden.

WO 2013/156 281 A1 lehrt die Behandlung von Superabsorbern mit Aluminiumglycinat.

WO 2010/108 875 A1, WO 2012/045 705 A1 und WO 2013/156 330 A1 lehren die Behandlung von Superabsorbern mit basischen Aluminiumsalzen wie basischem Aluminiumacetat oder Aluminiumlaktat.

WO 2009/080 611 A2 offenbart die Behandlung von Superabsorbern mit Mischungen von Aluminiumsalzen, von denen eines ein chelatisierendes Anion, beispielsweise Dicarboxylate oder Hydroxicarboxylate enthält, wobei Laktat besonders bevorzugt ist, und das andere ein schwach komplexierendes Anion, beispielsweise Chlorid, Nitrat, Sulfat, Hydrogensulfat, Carbonat, Hydrogencarbonat, Nitrat, Phosphat, Hydrogenphosphat, Dihydrogenphosphat oder Carboxylat, wobei Sulfat besonders bevorzugt ist. Die ältere Anmeldung beim Europäischen Patentamt mit dem Aktenzeichen 17 200 963.1 lehrt einen mit Aluminiumionen komplexierten Superabsorber, wobei die Aluminiumionen in Form einer wässrigen, Aluminiumionen enthaltenden Lösung aufgebracht werden, die sich dadurch auszeichnet, dass sie Aluminiumionen in einem Anteil im Bereich von 0,5 - 15 Gewichts-% (ggf. umgerechnet auf $Al^{3+}$) bezogen auf die Gesamtmasse der Lösung umfasst und weiterhin Anionen der Milchsäure (Laktat-Ionen) und der Phosphorsäure (Phosphat-Ionen) umfasst, wobei der molare Anteil der Laktat-Ionen im Bereich des 0,01 - 2,99-fachen bezogen auf den molaren Anteil an $Al^{3+}$ ist und der molare Anteil der Phosphat-Ionen im Bereich des 0,01 - 2,99-fachen bezogen auf den molaren Anteil an $Al^{3+}$ ist. Diese Lösungen werden durch Säurezugabe zu amorphem Aluminiumhydroxid hergestellt.

EP 233 067 A2 offenbart die Oberflächennachvernetzung von Superabsorbern mit Aluminiumsalzen, die in Gegenwart von Polyol und Wasser mit dem Superabsorber reagieren können. In einer Reihe von Aluminiumsalzen wird auch Aluminiumhydroxid genannt. Die Verwendung frisch gefällten Aluminiumhydroxid-Sols oder -Gels wird empfohlen.

Nach der Lehre von US 5 145 906 wird ungetrocknetes polymerisiertes Gel mit einem Oberflächennachvernetzer behandelt, die Nachvernetzungsreaktion findet im Zuge des Aufheizens zur Trocknung oder während der Trocknung statt. Aluminiumhydroxid ist einer der genannten möglichen Oberflächennachvernetzer. JP 09/124 879 A nennt ebenfalls in einer Reihe von Verbindungen auch Aluminiumhydroxid zu diesem Zweck, das jedoch wie die übrigen genannten, wasserlöslichen Verbindungen als Lösung aufgebracht werden soll. In EP 780 424 A1 werden die Hydroxide und Chloride einer Reihe von Metallen, darunter Aluminium, als Oberflächennachvernetzer genannt. US 5 684 106 nennt zu diesem Zweck Aluminiumsulfat, Natriumaluminat oder andere polyvalente Metallverbindungen. JP 3 121 934 B lehrt dazu die Verwendung von Polyaluminiumhydroxiden der Formel $[Al(OH)_3]_n \cdot AlCl_3$ mit n = 10 - 21).

WO 03/049 778 A1 lehrt, bei sowohl mit kovalenten Oberflächennachvernetzern als auch mehrwertigen Metallionen nachvernetzten Superabsorbern nach einer ersten Aufnahme von Flüssigkeit die Nachvernetzung mit Metallionen beispielsweise mit Komplexbildnern wieder zu lösen, um dadurch weitere Absorptionskapazität zu gewinnen. Aluminiumhydroxid wird in einer Reihe möglicher mehrwertiger Metallsalze als Nachvernetzer genannt.

Nach der Lehre von WO 2012/143 215 A1 wird dem Superabsorber eine Lösung eines neutralisierten mehrwertigen Metallsalzes zugesetzt, vorzugsweise eines Aluminiumsalzes, das aus einer Aluminiumverbindung und einer organischen Säure mit chelatbildendem Anion gebildet und wobei die Lösung mit Säure oder Base auf einen pH-Wert zwischen 5 und 9 neutralisiert wird. Die so mit einer Säure mit chelatbildendem Anion umgesetzte Aluminiumverbindung kann auch Aluminiumhydroxid sein. Die genannten organischen chelatbildenden Anionen bilden einen wasserlöslichen Komplex mit dem Metallion. WO 2013/72 311 A1 lehrt, zur Oberflächennachvernetzung einen Komplex aus einem Metallsalz wie etwa Aluminiumhydroxid und einem 2-Hydroxicarbonsäureamid zu verwenden.

WO 2014/167 036 A1, WO 2014/167 040 und WO 2014/168 858 A1 offenbaren wie schon die oben genannte EP 233 067 A2 die Aufbringung von frisch gefälltem Aluminiumhydroxid-Sol bei der Oberflächennachvernetzung.

US 2016/0 235 882 A1 lehrt, vor Aufbringung einer Lösung eines kovalenten Nachvernetzers Aluminiumhydroxidpulver in das Superabsorber-Grundpolymer einzumischen. Das Einmischen erfolgt vorzugsweise trocken, da bei Aufbringung von Suspensionen die Verbackungsneigung steigt.

Es besteht weiterhin die Aufgabe, andere oder sogar verbesserte, insbesondere permeable Superabsorber sowie möglichst vereinfachte oder verbesserte Verfahren zu ihrer Herstellung zu finden. Die Gebrauchseigenschaften des Superabsorbers, insbesondere seine Aufnahmefähigkeit für Flüssigkeit, auch unter Druck, sowie seine Quellkinetik, als volumetrische Absorption unter Druck "VAUL" quantifiziert, sollen dabei nicht oder zumindest nicht wesentlich beeinträchtigt werden. Weitere Aufgaben der Erfindung sind Verwendungen dieses Superabsorbers, wie Hygieneprodukte, die diesen Superabsorber enthalten und Verfahren zu deren Herstellung.

Gelöst wurde die Aufgabe durch ein Verfahren zur Herstellung eines Superabsorbers, umfassend

Polymerisation einer wässrigen Monomerlösung, die

a) mindestens ein ethylenisch ungesättigtes, säuregruppentragendes Monomer, das wahlweise zumindest teilweise als Salz vorliegt,
b) mindestens einen Vernetzer,
c) mindestens einen Initiator,
d) wahlweise ein oder mehrere mit den unter a) genannten Monomeren copolymerisierbare ethylenisch ungesättigte Monomere, und
e) wahlweise ein oder mehrere wasserlösliche Polymere enthält,

Trocknung des erhaltenen Polymerisats,

wahlweise Mahlung des getrockneten Polymerisats und Siebung des gemahlenen Polymerisats, und

wahlweise Oberflächennachvernetzung des getrockneten und gegebenenfalls gemahlenen und gesiebten Polymerisats,

das dadurch gekennzeichnet ist, dass man nach Trocknung, Mahlung oder Siebung und sofern eine Oberflächennachvernetzung durchgeführt wird vor, während oder nach dieser Oberflächennachvernetzung röntgenamorphes Aluminiumhydroxidpulver zugibt.

Die erfindungsgemäßen Superabsorber sind mit dem erfindungsgemäßen Verfahren erhältlich und werden vorzugsweise mit dem erfindungsgemäßen Verfahren hergestellt. Sie zeigen überraschend gute Permeabilität, ohne dass ihre übrigen Gebrauchseigenschaften wie CRC oder AUL wesentlich beeinträchtigt wären.

Weiterhin wurden Artikel zur Absorption von Flüssigkeiten gefunden, insbesondere Hygieneartikel zur Absorption von flüssigen Körperausscheidungen oder flüssigen Anteilen von Körperausscheidungen, die dadurch gekennzeichnet sind, dass sie den erfindungsgemäßen Superabsorber enthalten. Ferner wurden Verfahren zur Herstellung solcher Artikel zur Absorption von Flüssigkeiten gefunden, die dadurch gekennzeichnet sind, dass man bei der Herstellung dieser Artikel ihnen den erfindungsgemäßen Superabsorber zusetzt.

Dem erfindungsgemäßen Superabsorber wird bei seiner Herstellung im erfindungsgemäßen Verfahren röntgenamorphes Aluminiumhydroxid zugegeben.

Der Zusatz von mehrwertigen Metallsalzen zu Superabsorbern ist lange bekannt. Meistens werden Aluminiumsalze zugesetzt. Der Zusatz erfolgt üblicherweise im Zuge der Oberflächennachvernetzung mit einem kovalenten Oberflächennachvernetzer, also einer Verbindung, die kovalente Bindungen mit funktionellen Gruppen an der Oberfläche der Superabsorberpartikel ausbilden kann, typischerweise die Säuregruppen der üblichen Superabsorber auf Acrylsäurebasis. Letztlich führt der Zusatz von mehrwertigen Metallsalzen auch zu Vernetzungsstellen an der Oberfläche der Superabsorberpartikel, allerdings durch ionische Bindung. Oft wird die Behandlung von Superabsorbern mit mehrwertigen Metallsalzen im Zuge der Oberflächennachvernetzung als "Komplexierung" bezeichnet. "Komplexierung" ist streng genommen also lediglich ein eigener Begriff für den Sonderfall der Oberflächennachvernetzung, bei dem durch mehrwertige Metallionen ionische Bindungen zwischen mehreren polaren Gruppen an der Oberfläche der Superabsorberpartikel erzeugt werden und oft wird die Komplexierung auch unter "Oberflächennachvernetzung" diskutiert. Im Rahmen dieser Erfindung wird unter "Komplexierung" die Oberflächennachvernetzung mit mehrwertigen Metallionen, insbesondere Aluminium verstanden, um sie von der Oberflächennachvernetzung mit Nachvernetzern, die kovalente Bindungen mit polaren Gruppen an der Oberfläche der Superabsorberpartikel ausbilden, abzugrenzen.

Bei der Komplexierung wird das zugesetzte Metallsalz mit dem Superabsorber umgesetzt. Das hier erfindungsgemäß dem Superabsorber zugesetzte röntgenamorphe Aluminiumhydroxid wird deshalb nicht oder zumindest nicht vollständig als solches im Superabsorber erhalten bleiben. Wesentlich ist daher, dass der erfindungsgemäße Superabsorber mit röntgenamorphem Aluminiumhydroxid behandelt wurde - anders gesagt, dass es ihm zugesetzt wurde. Er kann, muss aber nicht zugesetztes röntgenamorphes Aluminiumhydroxid noch als solches enthalten.

Unter Aluminiumhydroxid wird hier Aluminiumtrihydroxid verstanden. Die Nomenklatur wird in der Literatur nicht einheitlich gehandhabt. Insbesondere wird Aluminiumhydroxid oft auch unter die Aluminiumoxidhydrate gezählt oder auch als hydratisiertes Aluminiumoxid bezeichnet. Aluminiumhydroxid wird in bekannter Weise aus Aluminiumsalzlösungen durch Basenzugabe oder aus Aluminatlösungen durch Säurezugabe, jeweils zum neutralen pH-Bereich, ausgefällt. Zur Herstellung von amorphem Aluminiumhydroxid ist schnell zu fällen, da bei langsamer Fällung aus Aluminatlösungen kristallines gamma-$Al(OH)_3$ ("Hydrargillit" oder "Gibbsit") oder aus Aluminiumsalzlösungen alpha-$Al(OH)_3$ ("Bayerit") entstehen. Mit der Zeit wandelt sich Bayerit in Hydrargillit um. Diese Hydroxide können langsam zu den Aluminiumoxidhydraten der Formel $Al(O)OH$ (Diaspor oder Böhmit) kondensieren, die gelegentlich auch als "Hydroxide" bezeichnet werden. Bei der Herstellung röntgenamorphen Aluminiumtrihydroxids wird daher nicht wie im Bayer-Prozess zur Herstellung von Aluminiumoxidhydraten mit kristallinem Aluminiumhydroxid angeimpft, sondern rasch gefällt und rasch getrocknet. Vorzugsweise wird frisch gefälltes Aluminiumhydroxidgel mittels Sprühtrocknung getrocknet. Chemie und Herstellung von Aluminiumhydroxid, -oxidhydrat und -oxid sind wohlbekannt, s. beispielsweise Holleman/Wiberg, Lehrbuch der Anorganischen Chemie, Walter de Gruyter & Co., Berlin, 103. Auflage 2016, ISBN 978-3-11-051854-2, Abschnitt 2.4 "Sauerstoffverbindungen des Aluminiums", oder Ullmann's Encyclopedia of Industrial Chemistry, Stichwort "Aluminum Oxide", Wiley-VCH Verlag Gmbh & Co. KGaA, Weinheim 2012, DOI 10.1002/14356007.a01_557. Einige strukturelle Unterschiede zwischen amorphem und kristallinem Aluminiumhydroxid auf atomarer/molekularer Ebene erklären T. Isobe et al., J. Colloid and Interface Science 261 (2003) 320-324, die im Übrigen auch einleitend weitere bekannte Methoden zur Herstellung von amorphem Aluminiumhydroxid nennen.

Röntgenamorphes Aluminiumhydroxid in Pulverform ist auch kommerziell erhältlich, beispielsweise als "Aluminiumhydroxid Trockengel", Artikelnummer 511066100, der Dr. Paul Lohmann GmbH KG, Hauptstraße 2, 31860 Emmerthal, Deutschland.

Röntgenamorph ist Aluminiumhydroxid dann, wenn es in einem Röntgen-Pulverdiffraktogramm keine Signale ("Linien") zeigt. Kristalline Substanzen beugen elektromagnetische Strahlung mit einer Wellenlänge in der Größenordnung der interatomaren Abstände - typischerweise wird Röntgenstrahlung verwendet - so dass ein Beugungsmuster der Strahlung gemessen werden kann. Bei ausreichend großen Einkristallen entstehen punktförmige Beugungsmuster, bei denen aus Lage und Intensität der Punkte die Kristallstruktur einschließlich Lage der Atome im Kristallgitter errechnet werden kann. An einem Pulver einer kristallinen Substanz kann ein Diffraktogramm gemessen werden, dass Maxima der gestreuten Röntgenstrahlung in Abhängigkeit vom Ablenkungswinkel als Signale ("Linien") zeigt, wobei die Halbhöhenbreite der Maxima mit der Größe der Kristalle im Pulver korreliert. All dies ist wohlbekannt, s. z. B. Ullmann's Encyclopedia of Industrial Chemistry, Stichwort "Structure Analysis by Diffraction", Wiley-VCH Verlag Gmbh & Co. KGaA, Weinheim 2012, DOI 10.1002/14356007.b05_341. Die Methodik ist etabliert und Geräte zur Messung von Pulverdiffraktogrammen sind kommerziell erhältlich.

Keine Signale im Diffraktogramm zeigen Pulver demnach dann, wenn sie keine Kristallstruktur aufweisen, die Atome oder Moleküle also völlig oder weitgehend ungeordnet sind, oder die Kristalle so klein sind, dass die Anzahl der zum Kristall angeordneten Elementarzellen im Kristall nicht zur fokussierten Beugung von Strahlung ausreicht und die Halbhöhenbreite der Maxima im Diffraktogramm so groß wird, dass sie in der Basislinie verschwinden und nur diffuse Strahlung gemessen werden kann. Entscheidend ist dabei die Größe der Kristalle im Pulver, nicht die Partikelgröße des Pulvers. In einem Pulverpartikel können zahlreiche kleinere Kristalle ("Primärkristalle" oder "Primärkristallite") agglomeriert oder auf sonstige Weise verbunden sein. Für das hier erfindungsgemäß verwendete röntgenamorphe Aluminiumhydroxid bedeutet das, dass die darin vorliegenden Primärkristallite - falls solche vorliegen - eine Größe von höchstens 2 nm aufweisen.

Das röntgenamorphe Aluminiumhydroxid kann im Gemisch mit kristallinem eingesetzt werden. In diesem Fall würden im Diffraktogramm des Gemisches dem kristallinen Anteil zuzuordnende Linien auftreten. Der Anteil an kristallinem Aluminiumhydroxid kann beispielsweise auch durch Eichung eines Pulverdiffraktogramms bestimmt werden, indem man einer Vergleichsprobe des Pulvers eine bekannte Menge kristallinen Aluminiumhydroxids zusetzt und die dadurch erzeugte Vergrößerung der Gesamtfläche aller Linien im Diffraktogramm in Beziehung zur Fläche aller Linien im Diffraktogramm der eigentlichen Probe setzt. All dies ist bekannt und etwa bei der Eichung von Chromatographen nicht anders.

Im Allgemeinen hat das gesamte zugesetzte Aluminiumhydroxid einen Anteil von mindestens 50 Gew.-% röntgenamorphem Aluminiumhydroxid, vorzugsweise von mindestens 70 Gew.% und in besonders bevorzugter Weise von mindestens 90 Gew.-%. Wünschenswert ist reines röntgenamorphes Aluminiumhydroxid ohne in einem Röntgendiffraktogramm auftretende kristalline Anteile oder zumindest solches Aluminiumhydroxid, das außer unvermeidlichen geringen kristallinen Anteilen (wie sie etwa bei der Alterung entstehen können) nur aus röntgenamorphem Aluminiumhydroxid besteht.

Erfindungsgemäß wird das röntgenamorphe Aluminiumhydroxidpulver im Allgemeinen in einer Menge von mindestens 0,01 Gew.-%, vorzugsweise mindestens 0,1 Gew.-% und in besonders bevorzugter Weise von mindestens 0,2 Gew.-%, noch bevorzugter von mindestens 0,3 Gew.-% sowie im Allgemeinen von höchstens 2 Gew.-%, vorzugsweise höchstens 1,5 Gew.-% und in besonders bevorzugter Weise von höchstens 1 Gew.-%, noch bevorzugter höchstens 0,7 Gew.-% zugegeben, jeweils bezogen auf den Superabsorber vor der Zugabe.

Erfindungsgemäß wird das röntgenamorphe Aluminiumhydroxid als Pulver nach Trocknung, Mahlung oder Siebung und sofern eine Oberflächennachvernetzung durchgeführt wird vor, während oder nach dieser Oberflächennachvernetzung zugegeben.

Im erfindungsgemäßen Verfahren wird das röntgenamorphe Aluminiumhydroxid demnach trocken in das Superabsorberpulver nach dessen Trocknung eingemischt, und, sofern nach Trocknung ein Siebschnitt des Superabsorbers gewonnen wird, in diesen Siebschnitt. Das kann mit jedem bekannten Mischapparat und -verfahren erfolgen und wird vorzugsweise in Mischern mit bewegten Mischwerkzeugen durchgeführt, wie beispielsweise Schneckenmischern, Scheiben-, Paddel- oder Schaufelmischern oder Mischern mit anderen Mischwerkzeugen. Geeignete Mischer sind beispielsweise als Pflugschar®-Mischer von Gebr. Lödige Maschinenbau GmbH, Elsener-Straße 7 - 9, 33102 Paderborn, Deutschland erhältlich.

Vorzugsweise wird nach der Einmischung des röntgenamorphen Aluminiumhydroxids der so erzeugte Superabsorber befeuchtet, also sein Wassergehalt erhöht. Dazu wird in einem eigenen Apparat oder bequemerweise im selben Mischer Wasser zugesetzt, etwa durch Aufsprühen über eine Düse, ebenso wie unten für das Aufsprühen einer Nachvernetzerlösung beschrieben. Im Allgemeinen wird dem Superabsorber dann Wasser in einer Menge von mindestens 0,1 Gew.-%, vorzugsweise von mindestens 0,5 Gew.-% und in besonders bevorzugter Form mindestens 1 Gew.-% sowie im Allgemeinen höchstens 10 Gew.-%, vorzugsweise höchstens 7 Gew.-% und in besonders bevorzugter Form höchstens 5 Gew.-%, jeweils bezogen auf den Superabsorber vor der Zugabe, zugesetzt.

Sofern der Superabsorber zum Zeitpunkt der Wasserzugabe nicht warm oder heiß ist (etwa nach einer vorhergehenden Trocknung), kann es vorteilhaft sein, bei dieser Nachbefeuchtung erwärmtes Wasser zugegeben. Im Allgemeinen wird Wasser mit einer Temperatur von mindestens 5 °C zugegeben, vorzugsweise mindestens 20 °C und in besonders bevorzugter Form mindestens 25 °C, beispielsweise Wasser einer Temperatur von 30 °C, 40 °C, 50 °C, 60 °C, 70 °C, 80 °C oder 90 °C. Es kann auch Wasserdampf verwendet werden.

Die Zugabe von röntgenamorphem Aluminiumhydroxid und wahlweise die von Wasser kann vor, während oder nach einer Oberflächennachvernetzung erfolgen. Falls die aufzugebende Oberflächennachvernetzerlösung Wasser enthält, kann bequemerweise die Zugabe von röntgenamorphem Aluminiumhydroxid vorher durchgeführt werden und Zugabe der Oberflächennachvernetzerlösung kann die wahlweise Zugabe von Wasser nach der Zugabe von röntgenamorphem Aluminiumhydroxid ersetzen.

Die zuzugebende Gesamtmenge röntgenamorphes Aluminiumhydroxid kann auch auf mehrere Zugabestellen oder -zeitpunkte aufgeteilt werden. Bevorzugt ist die Zugabe an einer Stelle und zu einem Zeitpunkt.

Im Übrigen ist das erfindungsgemäße Verfahren zur Herstellung von Superabsorbern bekannt. Es ist ein Verfahren zur wässrigen Lösungspolymerisation einer Monomermischung, die Folgendes umfasst:

a) mindestens ein ethylenisch ungesättigtes, mindestens eine Säuregruppe tragendes Monomer, das wahlweise zumindest teilweise als Salz vorliegt,
b) mindestens einen Vernetzer,

c) mindestens einen Initiator,
d) wahlweise ein oder mehrere mit den unter a) genannten Monomeren copolymerisierbare ethylenisch ungesättigte Monomere und
e) wahlweise ein oder mehrere wasserlösliche Polymere.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren oder ihre Salze, wie Acrylsäure, Methacrylsäure, Maleinsäure oder ihre Salze, Maleinsäureanhydrid und Itaconsäure oder ihre Salze. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomerlösung enthält vorzugsweise höchstens 250 Gew.-ppm, bevorzugt höchstens 130 Gew.-ppm, besonders bevorzugt höchstens 70 Gew.-ppm sowie bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a), wobei neutralisiertes Monomer a), d.h. ein Salz des Monomers a) rechnerisch als unneutralisiertes Monomer berücksichtigt wird. Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 530 438 A1 beschrieben, Di- und Triacrylate, wie in EP 547 847 A1, EP 559 476 A1, EP 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/32962 A2 beschrieben.

Bevorzugte Vernetzer b) sind Pentaerythrittriallylether, Tetraallyloxyethan, Methylenbismethacrylamid, 15-bis 20-fach ethoxiliertes Trimethylolpropantriacrylat, 15-20-fach ethoxiliertes Glycerintriacrylat, Polyethylenglykoldiacrylat mit zwischen 4 und 45 -CH$_2$CH$_2$O-Einheiten in der Molekülkette, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und steigt die Absorption unter Druck (AUL).

Als Initiatoren c) können sämtliche unter den Polymerisationsbedingungen in Radikale erzeugende Verbindungen eingesetzt werden, beispielsweise thermische Initiatoren, Redox-Initiatoren und/oder Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise auch ein Sulfonsäurederivat eingesetzt, beispielsweise ein Gemischen aus dem Natriumsalz der 2-Hydroxi-2-sulfinatoessigsäure, dem Dinatriumsalz der 2-Hydroxi-2-sulfonatoessigsäure und Natriumbisulfit, das beispielsweise von L. Brüggemann KG (Salzstraße 131, 74076 Heilbronn, Deutschland, www.brueggemann.com) unter den Bezeichnungen BRÜGGOLIT® FF6M oder BRÜGGOLIT® FF7, alternativ BRUGGO-LITE® FF6M oder BRUGGOLITE® FF7 erhältlich ist. oder das Dinatriumsalz der 2-Hydroxi-2-sulfonatoessigsäure, das beispielsweise von L. Brüggemann KG unter der Bezeichnung BLAN-COLEN® HP erhältlich ist. Die Initiatoren werden im Übrigen in üblichen Mengen verwendet. Die übliche Menge der reduzierenden Komponente eines Redoxinitiators liegt im Allgemeinen bei mindestens 0,00001 Gew.-%, vorzugsweise mindestens 0,0001 Gew.-% und besonders bevorzugt mindestens 0,001 Gew.-% sowie im Allgemeinen von höchstens 0,2 Gew.-% und vorzugsweise höchstens 0,1 Gew.-%, jeweils bezogen auf die Menge der Monomeren a) und d). Wird als reduzierende Komponente des Redoxinitiators jedoch allein Sulfonsäurederivat eingesetzt, liegt dessen zugesetzte Menge im Allgemeinen bei mindestens 0,001 Gew.-%, vorzugsweise mindestens 0,01 Gew.-% und besonders bevorzugt mindestens 0,03 Gew.-% sowie im Allgemeinen von höchstens 1,0 Gew.-%, vorzugsweise höchstens 0,3 Gew.-%, und besonders bevorzugt bei höchstens 0,2 Gew.-%, jeweils bezogen auf die Menge der Monomeren a) und d). Die übliche Menge der oxidierenden Komponente eines Redoxinitiators liegt im Allgemeinen bei 0,0001 Gew,-% und besonders bevorzugt mindestens 0,001 Gew.-% sowie im Allgemeinen von höchsten 2 Gew.-% und vorzugsweise höchstens 1,0 Gew.-%, jeweils bezogen auf die Menge der Monomeren a) und d). Die übliche Menge der thermischen Initiatoren liegt im Allgemeinen bei 0,01 Gew,-% und besonders bevorzugt mindestens 0,1 Gew.-% sowie im Allgemeinen von höchsten 2 Gew.-% und vorzugsweise höchstens 1,0 Gew.-%, jeweils bezogen auf die Menge der Monomeren a) und d). Die übliche Menge der Photoinitiatoren liegt im Allgemeinen bei 0,001 Gew,-% und besonders bevorzugt mindestens 0,01 Gew.-% sowie im Allgemeinen von höchsten 1,0 Gew.-% und vorzugsweise höchstens 0,2 Gew.-%, jeweils bezogen auf die Menge der Monomeren a) und d).

Mit den ethylenisch ungesättigten, säuregruppentragenden Monomeren a) copolymerisierbare ethylenisch ungesättigte Monomere d) sind beispielsweise Acrylamid, Methacrylamid, Hydroxiethylacrylat, Hydroxiethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat, Maleinsäure oder ihre Salze und Maleinsäureanhydrid.

Als wasserlösliche Polymere e) können Polyvinylalkohol, Polyvinylpyrrolidon, Stärke, Stärkederivate, modifizierte Cellulose, wie Methylcellulose oder Hydroxiethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. übersättigte Monomerlösungen einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Die Monomermischung kann weitere Komponenten enthalten. Beispiele in derartigen Monomermischungen verwendeter weiterer Komponenten sind etwa Chelatbildner, um Metallionen in Lösung zu halten oder anorganische Pulver, um die Steifigkeit des Superabsorbers im gequollenen Zustand zu erhöhen, oder rückgeführtes Unterkorn aus einer späteren Mahlung. Hier können alle bekannten Zusätze zur Monomermischung verwendet werden. Auch wenn im Zusammenhang mit der Monomermischung hier nur von "Lösung" die Rede ist, ist hiermit auch die Polymerisation einer Suspension mit gemeint, etwa wenn der Monomermischung unlösliche Bestandteile zugesetzt werden.

Die Säuregruppen der aus der Polymerisation erhaltenen Polymergele sind üblicherweise teilweise neutralisiert. Die Neutralisation wird vorzugsweise auf der Stufe der Monomeren durchgeführt, mit anderen Worten, es werden Salze der säuregruppentragenden Monomeren oder genaugenommen eine Mischung von säuregruppentragenden Monomeren und Salzen der säuregruppentragenden Monomeren ("teilneutralisierte Säure") als Komponente a) in die Polymerisation eingesetzt. Dies geschieht üblicherweise durch Einmischung des Neutralisationsmittels als wässrige Lösung oder bevorzugt auch als Feststoff in die zur Polymerisation vorgesehene Monomermischung oder bevorzugt in das säuregruppentragende Monomer oder eine Lösung davon. Der Neutralisationsgrad beträgt vorzugsweise von 25 bis 95 mol-%, besonders bevorzugt von 50 bis 80 mol-%, ganz besonders bevorzugt von 65 bis 72 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Es ist jedoch bevorzugt, die Neutralisation auf der Stufe des Monomeren durchzuführen. Mit anderen Worten: in einer ganz besonders bevorzugten Ausführungsform wird als Monomer a) ein Gemisch aus 25 bis 95 mol-%, besonders bevorzugt von 50 bis 80 mol-%, ganz besonders bevorzugt von 65 bis 75 mol-% Salz des säuregruppentragenden Monomeren und dem Rest zu 100 mol-% säuregruppentragendes Monomer eingesetzt. Dieses Gemisch ist beispielsweise ein Gemisch aus Natriumacrylat und Acrylsäure oder ein Gemisch aus Kaliumacrylat und Acrylsäure.

In einer bevorzugten Ausführungsform wird zur Neutralisation ein Neutralisationsmittel verwendet, dessen Gehalt an Eisen im Allgemeinen unter 10 Gew.-ppm, vorzugsweise unter 2 Gew.-ppm und in besonders bevorzugter Weise unter 1 Gew.-ppm liegt. Ebenso ist ein niedriger Gehalt an Chlorid sowie Anionen von Sauerstoffsäuren des Chlors erwünscht. Ein geeignetes Neutralisationsmittel ist beispielsweise die üblicherweise als "membrane grade" gehandelte 50 Gew.-%ige Natronlauge oder Kalilauge, noch reiner und ebenso geeignet, allerdings auch kostspieliger ist die üblicherweise als "amalgame grade" oder "mercury process" gehandelte 50 Gew.-%ige Natronlauge oder Kalilauge.

Verfahren zur Herstellung von Superabsorbern aus Monomermischungen wie der vorstehend beispielhaft beschriebenen sind grundsätzlich bekannt. Geeignete Polymerisationsreaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/38402 A1 beschrieben. Die Polymerisation auf dem Band wird beispielsweise in EP 955 086 A2, DE 38 25 366 A1 und US 6,241,928 beschrieben. Bei der Polymerisation in einem Bandreaktor entsteht wie auch bei der ebenso bekannten Polymerisation im Satzbetrieb oder im Rohrreaktor, wie beispielsweise in EP 445 619 A2 and DE 19 846 413 A1 beschrieben, ein Polymergel, das in einem weiteren Verfahrensschritt zerkleinert werden muss, beispielsweise in einem Fleischwolf, Extruder oder Kneter. Es können aber auch sphärische oder anders geformte Superabsorberpartikel durch Suspensions- oder Emulsionspolymerisation hergestellt werden, wie beispielsweise in EP 457 660 A1 beschrieben, oder durch Sprüh- oder Tropfenpolymerisationsverfahren hergestellt werden, wie beispielsweise in EP 348 180 A1, EP 816 383 A1, WO 96/404 27 A1, US 4 020 256, US 2002/0 193 546 A1, DE 35 19 013 A1, DE 10 2005 044 035 A1, WO 2007/093531 A1, WO 2008/086 976 A1 oder WO 2009/027 356 A1 beschrieben. Ebenso bekannt sind Verfahren, bei denen die Monomermischung auf ein Substrat wie beispielsweise eine Vliesbahn aufgebracht und polymerisiert wird, wie etwa in WO 02/94 328 A2 und WO 02/94 329 A1 beschrieben.

Dem Superabsorber oder auch der Monomermischung kann in bekannter Weise vor oder nach der Trocknung, vorzugsweise aber vor der Trocknung, wahlweise ein Sulfonsäurederivat zugesetzt werden, auch im Gemisch mit Sulfit oder Sulfinsäurederivat. Diese Gemische sind gängige Handelswaren und beispielsweise in der Form von Gemischen aus dem Natriumsalz der 2-Hydroxi-2-sulfinatoessigsäure, dem Dinatriumsalz der 2-Hydroxi-2-sulfonatoessigsäure und Natriumbisulfit von L. Brüggemann KG (Salzstraße 131, 74076 Heilbronn, Deutschland, www.brueggemann.com) unter den Bezeichnungen BRÜGGOLIT® FF6M oder BRÜGGOLIT® FF7, alternativ BRUGGOLITE® FF6M oder BRUGGO-LITE® FF7 erhältlich. Bevorzugt ist die Verwendung der Sulfonsäurederivate in Reinform. Auch diese sind gängige Handelswaren. So ist beispielsweise das Dinatriumsalz der 2-Hydroxi-2-sulfonatoessigsäure von L. Brüggemann KG (Salzstraße 131, 74076 Heilbronn, Deutschland, www.brueggemann.com) unter der Bezeichnung BLANCOLEN® HP erhältlich.

Das Sulfonsäurederivat wird im Allgemeinen in einer Menge von mindestens 0,0001 Gew.-%, vorzugsweise mindestens 0,001 Gew.-% und in besonders bevorzugter Form mindestens 0,025 Gew.-%, beispielsweise mindestens 0,05 Gew.-% oder mindestens 0,1 Gew.-% sowie im Allgemeinen höchstens 3 Gew.-%, in bevorzugter Form höchstens 2 Gew.-% und in besonders bevorzugter Form höchstens 0,5 Gew.-%, beispielsweise höchstens 0,35 Gew.-% oder 0,2 Gew.-%, jeweils auf das Gesamtgewicht des Superabsorbers bezogen, eingesetzt.

Dem Superabsorber oder auch der Monomermischung kann in bekannter Weise vor oder nach der Trocknung, vorzugsweise aber vor der Trocknung, ebenso wie das Sulfonsäurederivat zusätzlich zu diesem oder alleine wahlweise auch mindestens ein Phosphonsäurederivat zugesetzt werden. Besonders bevorzugt ist die Zugabe von bevorzugte Weise (1-Hydroxiethan-1,1-diyl)bisphosphonsäure ("Etidronsäure") oder eines Salzes davon, insbesondere dem Natriumsalz, dem Kaliumsalz, dem Dinatriumsalz, dem Dikaliumsalz oder dem Natrium-Kalium-Salz. Derartige Phosphonsäurederivate sind gängige Handelswaren und beispielsweise unter der Marke Modosol® (früher Cublen®) von Zschimmer & Schwarz Mohsdorf GmbH & Co KG, Chemnitztalstraße 1, 09217 Burgstädt, Deutschland erhältlich.

Das Phosphonsäurederivat wird im Allgemeinen in einer Menge von mindestens 0,01 Gew.-%, vorzugsweise mindestens 0,1 Gew.-% und besonders bevorzugt mindestens 0,2 Gew.-% sowie im Allgemeinen von höchstens 1,9 Gew.-%, vorzugsweise höchstens 1,3 Gew.-% und besonders bevorzugt höchstens 0,6 Gew.-%, jeweils bezogen auf die Gesamtmenge des wasserfreien Superabsorbers zugesetzt.

Das aus der wässrigen Lösungspolymerisation und gegebenenfalls nachträglicher Neutralisation erhaltene Polymergel wird dann vorzugsweise mit einem Bandtrockner getrocknet, bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt (Messmethode für den Restfeuchte- oder Wassergehalt siehe unten). Bei einer zu hohen Restfeuchte weist das getrocknete Polymergel eine zu niedrige Glasübergangstemperatur Tg auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung im Allgemeinen von 25 bis 90 Gew.-%, vorzugsweise von 30 bis 80 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein beheizbarer Mischer mit mechanischem Mischorgan wie beispielsweise ein Schaufeltrockner oder ein ähnlicher Trockner mit anders gestalteten Mischwerkzeugen verwendet werden. Wahlweise kann der Trockner unter Stickstoff oder einem anderen nicht-oxidierenden Inertgas oder zumindest unter verringertem Partialdruck des Sauerstoffs betrieben werden, um oxidative Vergilbungsvorgänge zu verhindern. Im Regelfall führt aber auch eine ausreichende Belüftung und Abführung des Wasserdampfes zu einem akzeptablen Produkt. Vorteilhaft hinsichtlich Farbe und Produktqualität ist in der Regel eine möglichst kurze Trocknungszeit.

Während der Trocknung verringert sich auch der Restmonomerengehalt in den Polymerpartikeln und letzte Reste des Initiators werden zerstört.

Das getrocknete Polymergel wird hiernach wahlweise - und bevorzugterweise - gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen eingesetzt werden können. Übergroße, oft im Inneren noch nicht getrocknete Gelklumpen sind gummielastisch, führen zu Problemen bei der Mahlung und werden vorzugsweise vor der Mahlung abgetrennt, was durch Windsichtung oder ein Sieb ("Schutzsieb" für die Mühle) in einfacher Weise erfolgen kann. Die Maschenweite des Siebs ist angesichts der verwendeten Mühle so zu wählen, dass möglichst keine Störungen durch übergroße, gummielastische Partikel auftreten.

Zu große, nicht ausreichend fein gemahlene Superabsorberpartikel sind bei ihrer überwiegenden Verwendung, in Hygieneprodukten wie Windeln, als grobe Partikel fühlbar, sie senken auch die mittlere Anquellgeschwindigkeit des Superabsorbers Beides ist unerwünscht. Vorteilhafterweise werden daher grobkörnige Polymerpartikel aus dem Produkt abgetrennt. Dies erfolgt durch übliche Klassierverfahren, beispielsweise Windsichtung oder durch Siebung durch ein Sieb mit einer Maschenweite von höchstens 1000 $\mu$m, vorzugsweise höchstens 900 $\mu$m, besonders bevorzugt höchstens 850 $\mu$m und ganz besonders bevorzugt höchstens 800 $\mu$m. Beispielsweise werden Siebe mit 700 $\mu$m, 650 $\mu$m oder 600 $\mu$m Maschenweite verwendet. Die abgetrennten grobkörnigen Polymerpartikel ("Überkorn") können zur Kostenoptimierung dem Mahl- und Siebkreislauf wieder zugeführt oder separat weiterverarbeitet werden.

Polymerpartikel mit zu niedriger Partikelgröße senken die Permeabilität (SFC). Vorteilhafterweise werden daher bei dieser Klassierung auch feinkörnige Polymerpartikel abgetrennt. Dies kann, falls gesiebt wird, bequem durch ein Sieb mit einer Maschenweite von höchstens 300 $\mu$m, vorzugsweise höchstens 200 $\mu$m, in besonders bevorzugter Weise höchstens 150 $\mu$m und in ganz besonders bevorzugter Weise höchstens 100 $\mu$m erfolgen. Die abgetrennten feinkörnigen Polymerpartikel ("Unterkorn" oder "fines") können zur Kostenoptimierung beliebig dem Monomerstrom, dem polymerisierenden Gel, oder dem auspolymerisierten Gel vor der Trocknung des Gels wieder zugeführt werden.

Die mittlere Partikelgröße der als Produktfraktion abgetrennten Polymerpartikel beträgt im Allgemeinen mindestens 200 $\mu$m, bevorzugt mindestens 250 $\mu$m und in bevorzugter Form mindestens 300 $\mu$m sowie im Allgemeinen höchstens 600 $\mu$m und in bevorzugter Weise höchstens 500 $\mu$m. Der Anteil an Partikeln mit einer Partikelgröße von mindestens 150 $\mu$m beträgt im Allgemeinen mindestens 90 Gew.-%, bevorzugterweise mindestens 95 Gew.-% und in besonders bevorzugter Weise mindestens 98 Gew.-%. Der Anteil an Partikeln mit einer Partikelgröße von höchstens 850 $\mu$m, beträgt im Allgemeinen mindestens 90 Gew.-%, bevorzugterweise mindestens 95 Gew.-% und in besonders bevorzugter Weise mindestens 98 Gew.-%.

Bei manchen anderen bekannten Herstellverfahren für Superabsorber, insbesondere bei Suspensionspolymerisation, Sprüh- oder Vertropfungspolymerisation wird durch die Wahl der Verfahrensparameter die Partikelgrößenverteilung vorgegeben. Bei diesen Verfahren entsteht direkt partikulärer Superabsorber der gewünschten Partikelgröße, so dass Mahl- und Siebschritte oft entfallen können. In manchen Verfahren (insbesondere bei Sprüh- oder Vertropfungspolymerisation) kann oft auch ein eigener Trocknungsschritt entfallen.

Das so hergestellte Polymer hat superabsorbierende Eigenschaften und fällt unter den Begriff "Superabsorber". Seine CRC ist typischerweise vergleichsweise hoch, seine AUL oder SFC dagegen vergleichsweise niedrig. Ein derartiger, nicht oberflächennachvernetzter Superabsorber wird zur Unterscheidung von einem daraus hergestellten oberflächennachvernetzten Superabsorber oft "Grundpolymer" oder "Basispolymer" genannt.

Findet keine Oberflächennachvernetzung statt oder gibt man das röntgenamorphe Aluminiumhydroxid vor der Oberflächennachvernetzung zu, gibt man es nun wie oben beschrieben diesem Grundpolymer zu.

Das Grundpolymer wird wahlweise oberflächennachvernetzt. Oberflächennachvernetzer für Superabsorber und Verfahren zur Oberflächennachvernetzung von Superabsorbern sind wohlbekannt. Geeignete Nachvernetzer sind Verbindungen, die Gruppen enthalten, die mit mindestens zwei funktionellen Gruppen der Superabsorberpartikel Bindungen bilden können. Bei den auf dem Markt vorherrschenden Superabsorbern auf Acrylsäure/Natriumacrylat-Basis sind geeignete Oberflächennachvernetzer Verbindungen, die Gruppen enthalten, die mit mindestens zwei Carboxylatgruppen Bindungen bilden können. Statt "Oberflächennachvernetzer" oder "Oberfächennachvernetzung" wird oft auch nur "Nachvernetzer" oder "Nachvernetzung" verwendet.

Bevorzugte Oberflächennachvernetzer sind Di- oder Triglycidylverbindungen wie beispielsweise Glycidylether, etwa Ethylenglykoldiglycidylether, Glyzerindi- oder -triglycidylether.

Bevorzugte Oberflächennachvernetzer sind auch 2-Oxazolidone, wie 2-Oxazolidon und N-(2-Hydroxiethyl)-2-oxazolidon, N-Methyl-2-Oxazolidon, N-Acyl-2-oxazolidone, wie N-Acetyl-2-oxazolidon, 2-Oxotetrahydro-1,3-oxazin, bicyclische Amidacetale, wie 5-Methyl-1-aza-4,6-dioxa-bicyclo[3.3.0]octan, 1-Aza-4,6-dioxa-bicyclo[3.3.0]octan und 5-Isopropyl-1-aza-4,6-dioxa-bicyclo [3.3.0] octan, Bis-2-oxazolidone und Poly-2-oxazolidone. Unter diesen sind besonders bevorzugt 2-Oxazolidon, N-Methyl-2-oxazolidon, N-(2-Hydroxiethyl)-2-oxazolidon und N-Hydroxipropyl-2-oxazolidon.

Weitere bevorzugte Nachvernetzer sind 1,3-Propandiol, 1,5-Pentandiol, 1,6-Hexandiol und 1,7-Heptandiol, 1,3-Butandiol, 1,8-Octandiol, 1,9-Nonandiol und 1,10-Decandiol. Unter diesen sind besonders bevorzugt diejenigen, die sich bei 23°C zu mindestens 30 Gew.-%, bevorzugt zu mindestens 40 Gew.-%, besonders bevorzugt zu mindestens 50 Gew.-%, ganz besonders bevorzugt mindestens zu 60 Gew.-%, in Wasser lösen, wie beispielsweise 1,3-Propandiol und 1,7-Heptandiol. Noch mehr bevorzugt sind solche, die bei 25°C flüssig sind.

Weitere bevorzugte Nachvernetzer sind Butan-1,2,3-triol, Butan-1,2,4-triol, Glyzerin, Trimethylolpropan, Trimethylolethan, Pentaerythrit, pro Molekül 1- bis 3-fach ethoxyliertes Glyzerin, Trimethylolethan oder Trimethylolpropan und pro Molekül 1- bis 3-fach propoxyliertes Glyzerin, Trimethylolethan oder Trimethylolpropan. Weiterhin bevorzugt sind 2-fach ethoxyliertes oder propoxyliertes Neopentylglykol. Besonders bevorzugt sind 2-fach und 3-fach ethoxyliertes Glyzerin, Neopentylglykol, 2-Methyl-1,3-propandiol und Trimethylolpropan. Unter diesen besonders bevorzugt sind diejenigen, die bei 23 °C eine Viskosität von weniger als 3000 mPas, vorzugsweise weniger als 1500 mPas, bevorzugt weniger als 1000 mPas, besonders bevorzugt weniger als 500 mPas, ganz besonders bevorzugt weniger als 300 mPas, aufweisen.

Weitere bevorzugte Nachvernetzer sind Ethylencarbonat und Propylencarbonat.

Ein weiterer bevorzugter Nachvernetzer ist 2,2'-Bis(2-oxazolin).

Ebenso bevorzugte Nachvernetzer sind Oxetane, insbesondere 3-Ethyl-oxetan-3-methanol oder 3,3'-[Oxibis(methylen)]bis[(3-ethyl)oxetan].

Diese bevorzugten Nachvernetzer minimieren Neben- und Folgereaktionen, die zu flüchtigen und damit übelriechenden Verbindungen führen. Die mit den bevorzugten Nachvernetzern hergestellten Superabsorber sind daher auch im angefeuchteten Zustand geruchsneutral.

Es kann ein einzelner Nachvernetzer verwendet werden oder beliebige Gemische verschiedener Nachvernetzer.

Der Nachvernetzer wird im Allgemeinen in einer Menge von mindestens 0,001 Gew.-%, vorzugsweise von mindestens 0,02 Gew.-%, in besonders bevorzugter Form von mindestens 0,05 Gew.-% sowie im Allgemeinen höchstens 2 Gew.-%, vorzugsweise höchstens 1 Gew.-%, in besonders bevorzugter Form höchstens 0,3 Gew.-%, beispielsweise höchstens 0,15 Gew.-% oder höchstens 0,095 Gew.-% eingesetzt, jeweils auf die Masse des damit beaufschlagten Grundpolymeren (beispielsweise der betreffenden Siebfraktion) bezogen.

Die Nachvernetzung wird üblicherweise so durchgeführt, dass eine Lösung des Nachvernetzers auf die getrockneten Grundpolymerpartikel aufgesprüht wird. Im Anschluss an das Aufsprühen werden die mit Nachvernetzer beschichteten Polymerpartikel thermisch getrocknet, wobei die Nachvernetzungsreaktion sowohl vor als auch während der Trocknung stattfinden kann. Falls Oberflächennachvernetzer mit polymerisierbaren Gruppen verwendet werden, kann die Oberflächennachvernetzung auch durch radikalisch induzierte Polymerisation solcher Gruppen mittels gängiger Radikalbildner oder auch mittels energiereicher Strahlung wie beispielsweise UV-Licht erfolgen. Dies kann parallel oder anstatt der Verwendung von Nachvernetzern erfolgen, die kovalente oder ionische Bindungen zu funktionellen Gruppen an der Oberfläche der Grundpolymerpartikel ausbilden.

Das Aufsprühen der Nachvernetzerlösung wird vorzugsweise in Mischern mit bewegten Mischwerkzeugen, wie Schneckenmischern, Scheiben-, Paddel- oder Schaufelmischern oder Mischern mit anderen Mischwerkzeugen durchgeführt. Besonders bevorzugt sind jedoch Vertikalmischer. Es ist aber auch möglich die Nachvernetzerlösung in einem Wirbelbett aufzusprühen.

Geeignete Mischer sind beispielsweise als Pflugschar®-Mischer von Gebr. Lödige Maschinenbau GmbH, Elsener-Straße 7 - 9, 33102 Paderborn, Deutschland, oder als Schugi® Flexomix®-Mischer, Vrieco-Nauta®-Mischer oder Turbulizer®-Mischer von Hosokawa Micron BV, Gildenstraat 26, 7000 AB Doetinchem, Niederlande, erhältlich.

Die einsetzbaren Sprühdüsen unterliegen keiner Beschränkung. Geeignete Düsen und Zerstäubungssysteme sind beispielsweise in den folgenden Literaturstellen beschrieben: Zerstäuben von Flüssigkeiten, Expert-Verlag, Bd.

660, Reihe Kontakt & Studium, Thomas Richter (2004) sowie in Zerstäubungstechnik, Springer-Verlag, VDI-Reihe, Günter Wozniak (2002). Einsetzbar sind mono- und polydisperse Sprühsysteme. Unter den polydispersen Systemen sind Einstoff-Druckdüsen (strahl- oder lamellenbildend), Rotationszerstäuber, Zweistoffzerstäuber, Ultraschallzerstäuber und Pralldüsen geeignet. Bei den Zweistoffzerstäubern kann die Mischung der Flüssigkeits- mit der Gasphase sowohl innenliegend als auch außenliegend erfolgen. Das Sprühbild der Düsen ist unkritisch und kann jede beliebige Form annehmen, beispielsweise Rundstrahl-, Flachstrahl-, Weitwinkel-Rundstrahl- oder Kreisring-Spritzbild. Vorteilhaft ist die Verwendung eines nicht-oxidierenden Gases, falls Zweistoffzerstäuber eingesetzt werden, besonders bevorzugt sind Stickstoff, Argon oder Kohlendioxid. Derartigen Düsen kann die zu versprühende Flüssigkeit unter Druck zugeführt werden. Die Zerteilung der zu versprühenden Flüssigkeit kann dabei dadurch erfolgen, dass sie nach Erreichen einer bestimmten Mindestgeschwindigkeit in der Düsenbohrung entspannt wird. Ferner können für den erfindungsgemäßen Zweck auch Einstoffdüsen, wie beispielsweise Schlitzdüsen oder Drallkammern (Vollkegeldüsen) verwendet werden (beispielsweise von Düsen-Schlick GmbH, DE, oder von Spraying Systems Deutschland GmbH, DE). Derartige Düsen sind auch in der EP 0 534 228 A1 und der EP 1 191 051 A2 beschrieben.

Die Nachvernetzer werden typischerweise als wässrige Lösung eingesetzt. Wird ausschließlich Wasser als Lösungsmittel verwendet, so wird der Nachvernetzerlösung oder bereits dem Grundpolymer vorteilhafterweise ein Tensid oder Deagglomerisationshilfsmittel zugesetzt. Dadurch wird das Benetzungsverhalten verbessert und die Verklumpungsneigung vermindert.

Alle anionischen, kationischen, nichtionischen und amphoteren Tenside sind als Deagglomerationshilfsmittel geeignet, bevorzugt sind jedoch aus Hautverträglichkeitsgründen nicht-ionische und amphotere Tenside. Das Tensid kann auch Stickstoff enthalten. Beispielsweise werden Sorbitanmonoester, wie Sorbitanmonococoat und Sorbitanmonolaurat, oder ethoxylierte Varianten davon, wie beispielsweise Polysorbat 20®, zugesetzt. Weitere geeignete Deagglomerationshilfsmittel stellen die ethoxylierten und alkoxylierten Derivate des 2-Propylheptanols dar, die unter den Marken Lutensol XL® und Lutensol XP® vertrieben werden (BASF SE, Carl-Bosch-Straße 38, 67056 Ludwigshafen, Deutschland).

Das Deagglomerationshilfsmittel kann getrennt dosiert oder der Nachvernetzerlösung zugesetzt werden. Vorzugsweise wird das Deagglomerationshilfsmittel der Nachvernetzerlösung einfach zugesetzt.

Die Einsatzmenge des Deagglomerationshilfsmittels bezogen auf Grundpolymer beträgt beispielsweise 0 bis 0,1 Gew.-%, vorzugsweise 0 bis 0,01 Gew.-%, besonders bevorzugt 0 bis 0,002 Gew.-%. Vorzugsweise wird das Deagglomerationshilfsmittel so dosiert, dass die Oberflächenspannung eines wässrigen Extrakts des gequollenen Grundpolymers und/oder des gequollenen nachvernetzten wasserabsorbierenden Polymeren bei 23 °C mindestens 0,060 N/m, vorzugsweise mindestens 0,062 N/m, besonders bevorzugt mindestens 0,065 N/m, und vorteilhaft höchstens 0,072 N/m beträgt.

Die wässrige Nachvernetzerlösung kann neben dem mindestens einen Nachvernetzer auch noch ein Cosolvens enthalten. Über den Gehalt an nichtwässrigem Lösungsmittel bzw. Gesamtlösungsmittelmenge kann die Eindringtiefe des Nachvernetzers in die Polymerpartikel eingestellt werden. Technisch gut geeignete Cosolventien sind C1-C6-Alkohole, wie Methanol, Ethanol, n-Propanol, Isopropanol, n-Butanol, sec-Butanol, tert.-Butanol oder 2-Methyl-1-propanol, $C_2$-$C_5$-Diole, wie Ethylenglykol, 1,2-Propylenglykol oder 1,4-Butandiol, Ketone, wie Aceton, oder Carbonsäureester, wie Essigsäureethylester. Nachteilig an einigen dieser Cosolventien ist, dass sie typische Eigengerüche aufweisen.

Das Cosolvens selbst ist unter den Reaktionsbedingungen idealerweise kein Nachvernetzer. Es kann jedoch im Grenzfall und abhängig von Verweilzeit und Temperatur dazu kommen, dass das Cosolvens teilweise zur Vernetzung beiträgt. Dies ist insbesondere dann der Fall, wenn der Nachvernetzer relativ träge ist und daher auch selbst sein Cosolvens bilden kann, wie beispielsweise bei Einsatz cyclischer Carbonate, Diole oder Polyole. Solche Nachvernetzer können im Gemisch mit reaktiveren Nachvernetzern auch in der Funktion als Cosolvens eingesetzt werden, da die eigentliche Nachvernetzungsreaktion dann bei niedrigeren Temperaturen und/oder kürzeren Verweilzeiten als in Abwesenheit des reaktiveren Vernetzers durchgeführt werden kann. Da das Cosolvens in relativ großen Mengen verwendet wird und auch teilweise im Produkt verbleibt, darf es nicht toxisch sein.

Im erfindungsgemäßen Verfahren eignen sich die oben genannten Diole, Polyole, sowie die cyclischen Carbonate auch als Cosolventien. Diese Funktion erfüllen sie in Gegenwart eines reaktiveren Nachvernetzers und/oder einer Di- oder Triglycidylverbindung. Bevorzugte Cosolventien im erfindungsgemäßen Verfahren sind jedoch insbesondere die genannten Diole, insbesondere, wenn die Hydroxigruppen sterisch durch Nachbargruppen an einer Reaktion behindert werden. Solche Diole eignen sich zwar prinzipiell auch als Nachvernetzer, erfordern dazu jedoch deutlich höhere Reaktionstemperaturen oder gegebenenfalls höhere Einsatzmengen als sterisch ungehinderte Diole.

Besonders bevorzugte Kombinationen aus wenig reaktivem Nachvernetzer als Cosolvens und reaktivem Nachvernetzer sind Kombinationen von genannten mehrwertigen Alkoholen, Diolen und Polyolen mit den genannten Amidacetalen oder Carbamaten. Geeignete Kombinationen sind beispielsweise 2-Oxazolidon/1,2-Propandiol und N-(2-Hydroxiethyl)-2-oxazolidon/1,2-Propandiol sowie Ethylenglykoldiglycidylether/1,2-Propandiol. Ganz besonders bevorzugte Kombinationen sind 2-Oxazolidon/1,3-Propandiol und N-(2-Hydroxiethyl)-2-oxazolidon/1,3-Propandiol. Weiterhin bevorzugte Kombinationen sind solche mit Ethylenglykoldiglycidylether oder Glyzerindi- oder -triglycidylether mit folgenden Lösemitteln, Cosolventien oder Covernetzern: Isopropanol, 1,3-Propandiol, 1,2-Propylenglykol oder Gemischen davon. Weiterhin bevorzugte Kombinationen sind solche mit 2-Oxazolidon oder (2-Hydroxiethyl)-2-oxazolidon in folgenden Lösemitteln, Cosolventien oder Covernetzern: Isopropanol, 1,3-Propandiol, 1,2-Propylenglykol, Ethylencarbonat, Propylencarbonat oder Gemischen davon.

Häufig beträgt die Konzentration des Cosolvens in der wässrigen Nachvernetzerlösung, von 15 bis 50 Gew.-%, vorzugsweise von 15 bis 40 Gew.-%, besonders bevorzugt von 20 bis 35 Gew.-%, bezogen auf die Nachvernetzerlösung. Bei Cosolventien, die mit Wasser nur begrenzt mischbar sind, wird man vorteilhaft die wässrige Nachvernetzerlösung so einstellen, dass nur eine Phase vorliegt, gegebenenfalls durch Erniedrigung der Konzentration des Cosolvens.

In einer bevorzugten Ausführungsform wird kein Cosolvens eingesetzt. Der Nachvernetzer wird dann nur als Lösung in Wasser angewandt, gegebenenfalls unter Zusatz eines Deagglomerationshilfsmittels.

Die Konzentration des mindestens einen Nachvernetzers in der wässrigen Nachvernetzerlösung, beträgt typischerweise 1 bis 20 Gew.-%, vorzugsweise 1,5 bis 10 Gew.-%, besonders bevorzugt 2 bis 5 Gew.-%, bezogen auf die Nachvernetzerlösung.

Die Gesamtmenge der Nachvernetzerlösung bezogen auf Grundpolymer beträgt üblicherweise von 0,3 bis 15 Gew.-%, vorzugsweise von 2 bis 6 Gew.-%.

Die eigentliche Oberflächennachvernetzung durch Reaktion des Oberflächennachvernetzers mit funktionellen Gruppen an der Oberfläche der Grundpolymerpartikel wird meist durch Erwärmung des mit Oberflächennachvernetzerlösung benetzten Grundpolymers durchgeführt, üblicherweise "Trocknung" genannt (aber nicht mit der oben beschriebenen Trocknung des Polymergels aus der Polymerisation zu verwechseln, bei der typischerweise sehr viel mehr Flüssigkeit zu entfernen ist). Die Trocknung kann im Mischer selbst erfolgen, durch Beheizung des Mantels, durch Wärmeaustauschflächen oder Einblasen warmer Gase. Gleichzeitiges Versetzen des Superabsorbers mit Oberflächennachvernetzer und Trocknen kann beispielsweise in einem Wirbelschichttrockner erfolgen. Die Trocknung wird aber meist in einem nachgeschalteten Trockner, wie beispielsweise einem Hordentrockner, einem Drehrohrofen, ein Paddel- oder Scheibentrockner oder einer beheizbaren Schnecke durchgeführt. Geeignete Trockner sind beispielsweise als Solidair® oder Torusdisc®-Trockner von Bepex International LLC, 333 N.E. Taft Street, Minneapolis, MN 55413, U.S.A., oder als Paddel- oder Schaufeltrockner oder auch als Fließbetttrockner von Nara Machinery Co., Ltd., Zweigniederlassung Europa, Europaallee 46, 50226 Frechen, Deutschland erhältlich.

Es ist möglich die Polymerpartikel zur Trocknung und Durchführung der Oberflächennachvernetzung über Kontaktflächen in einem nachgeschalteten Trockner zu beheizen, oder über zugeführtes warmes Inertgas, oder über eine Mischung eines oder mehrerer Inertgase mit Wasserdampf, oder nur mit Wasserdampf allein. Bei Zufuhr der Wärme über Kontaktflächen ist es möglich die Reaktion bei leichtem oder vollständigem Unterdruck unter Inertgas durchzuführen. Bei Verwendung von Wasserdampf zum direkten Beheizen der Polymerpartikel ist es erfindungsgemäß wünschenswert den Trockner bei Normaldruck oder Überdruck zu betreiben. In diesem Fall kann es sinnvoll sein den Nachvernetzungsschritt in einen Aufheizschritt mit Wasserdampf und einen Reaktionsschritt unter Inertgas aber ohne Wasserdampf aufzuspalten. Dies kann in einem oder mehreren Apparaten realisiert werden. Erfindungsgemäß können die Polymerpartikel schon im Nachvernetzungsmischer mit Wasserdampf aufgeheizt werden. Das eingesetzte Grundpolymer kann aus vorhergehenden Prozessschritten noch eine Temperatur von 10 bis 120 °C aufweisen, die Nachvernetzerlösung kann eine Temperatur von 0 bis 70 °C aufweisen. Insbesondere kann die Nachvernetzerlösung zur Verminderung der Viskosität erwärmt werden.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten. Typischerweise wird die Trocknung so geführt, dass der Superabsorber einen Restfeuchtegehalt von im Allgemeinen mindestens 0,1 Gew.-%, vorzugsweise mindestens 0,2 Gew.-% und in besonders bevorzugter Form mindestens 0,5 Gew.-% sowie im Allgemeinen höchstens 15 Gew.-%, vorzugsweise höchstens 10 Gew.-% und in besonders bevorzugter Form höchstens 8 Gew.-% aufweist.

Die Nachvernetzung kann unter normalen atmosphärischen Bedingungen stattfinden. Normale atmosphärische Bedingungen bedeutet, dass keine technischen Vorkehrungen getroffen werden, um den Partialdruck oxidierender Gase wie den des atmosphärischen Sauerstoffs im Apparat, in dem die Nachvernetzungsreaktion überwiegend stattfindet (dem "Nachvernetzungsreaktor", typischerweise der Trockner) zu verringern. Es ist jedoch bevorzugt, die Nachvernetzungsreaktion unter verringertem Partialdruck oxidierender Gase durchzuführen. Oxidierende Gase sind Stoffe, die bei 23 °C einen Dampfdruck von mindestens 1013 mbar aufweisen und in Verbrennungsvorgängen als Oxidationsmittel wirken, beispielsweise Sauerstoff, Stickoxid und Stickstoffdioxid, insbesondere Sauerstoff. Vorzugsweise beträgt der Partialdruck oxidierender Gase dabei weniger als 140 mbar, bevorzugt weniger als 100 mbar, besonders bevorzugt weniger als 50 mbar, ganz besonders bevorzugt weniger als 10 mbar. Wird die thermische Nachvernetzung bei Umgebungsdruck, d. h. bei einem Gesamtdruck um 1013 mbar, durchgeführt, so wird der Gesamtpartialdruck der oxidierenden Gase über deren Volumenanteil festgelegt. Der Anteil der oxidierenden Gase beträgt dabei vorzugsweise weniger als 14 Vol.-%, bevorzugt weniger als 10 Vol.-%, besonders bevorzugt weniger als 5 Vol.-%, ganz besonders bevorzugt weniger als 1 Vol.-%.

Die Nachvernetzung kann unter vermindertem Druck durchgeführt werden, d. h. bei einem Gesamtdruck von weniger als 1.013 mbar. Der Gesamtdruck beträgt typischerweise weniger als 670 mbar, vorzugsweise weniger als 480 mbar, besonders bevorzugt weniger als 300 mbar, ganz besonders bevorzugt weniger als 200 mbar. Werden Trocknung und Nachvernetzung unter Luft mit einem Sauerstoffgehalt von 20,8 Vol.-% durchgeführt, so betragen die zu den obengenannten Gesamtdrücken korrespondierenden Sauerstoffpartialdrücke 139 mbar (670 mbar), 100 mbar (480 mbar), 62 mbar (300 mbar) und 42 mbar (200 mbar), wobei die jeweiligen Gesamtdrücke in den Klammern stehen. Eine andere Möglichkeit, den Partialdruck oxidierender Gase zu senken, ist die Einleitung von nicht oxidierenden Gasen, insbesondere Inertgasen in den zur Nachvernetzung verwendeten Apparat. Geeignete Inertgase sind bei der Nachvernetzungstemperatur und gegebenem Druck im Nachvernetzungstrockner gasförmig vorliegende Stoffe, die unter diesen Bedingungen nicht oxidierend auf die Bestandteile der trocknenden Polymerpartikel wirken, beispielsweise Stickstoff, Kohlendioxid, Argon, Wasserdampf, wobei Stickstoff bevorzugt ist. Die Inertgasmenge beträgt im Allgemeinen von 0,0001 bis 10 $m^3$, bevorzugt 0,001 bis 5 $m^3$, besonders bevorzugt 0,005 bis 1 $m^3$, und ganz besonders bevorzugt 0,005 bis 0,1 $m^3$, bezogen auf 1 kg Superabsorber.

Im erfindungsgemäßen Verfahren kann das Inertgas, wenn es nicht Wasserdampf enthält, über Düsen in den Nachvernetzungstrockner eingeblasen werden, besonders bevorzugt wird das Inertgas aber bereits im oder kurz vor dem Mischer, indem der Superabsorber mit Oberflächennachvernetzer versetzt wird, über Düsen dem Polymerpartikelstrom zugegeben.

Selbstverständlich können aus dem Trockner abgeführte Dämpfe von Cosolventien außerhalb des Trockners wieder kondensiert und gegebenenfalls rezykliert werden.

Eine Möglichkeit, das röntgenamorphe Aluminiumhydroxid während der Oberflächennachvernetzung zuzugeben, ist das Einmischen während der Oberflächennachvernetzungsreaktion durch Zugabe in einen für die Oberflächennachvernetzungsreaktion verwendeten Apparat, sofern in diesem Apparat der Inhalt durchmischt wird. Geeignet sind etwa die oben dazu genannten Apparate mit Ausnahme des Hordentrockners. Vorzugsweise werden dazu Paddel- und Schaufeltrockner verwendet.

Wahlweise werden auf die Oberflächen des erfindungsgemäßen Superabsorbers vor, während oder nach der Nachvernetzung zusätzlich zu den genannten, kovalente Bindungen mit Carboxigruppen des Superabsorbers ausbildenden organischen Nachvernetzern und zusätzlich zum röntgenamorphen Aluminiumhydroxid mehrwertige Metallionen aufgebracht oder, falls keine Oberflächennachvernetzung mit einem der genannten organischen Nachvernetzer durchgeführt wird, anstatt dieser. Wie oben bereits gesagt, ist dieses Aufbringen von mehrwertigen Metallionen im Prinzip eine (gegebenenfalls zusätzliche) Oberflächennachvernetzung durch ionische, nicht kovalente Bindungen und wird im Rahmen dieser Erfindung zur Unterscheidung von Oberflächennachvernetzung mittels kovalenter Bindungen als "Komplexierung" mit den betreffenden Metallionen bezeichnet.

Dieses Aufbringen von polyvalenten Kationen erfolgt üblicherweise durch Aufsprühen von Lösungen zwei- oder mehrwertiger Kationen, meist zwei-, drei- oder vierwertiger Metallkationen, aber auch polyvalenter Kationen wie formal ganz oder teilweise aus Vinylaminmonomeren aufgebauter Polymere wie teilweise oder vollständig hydrolysiertes Polyvinylamid (sogenanntes "Polyvinylamin"), dessen Amingruppen stets - auch bei sehr hohen pH-Werten - teilweise zu Ammoniumgruppen protoniert vorliegen. Beispiele verwendbarer zweiwertiger Metallkationen sind insbesondere die zweiwertigen Kationen von Metallen der Gruppen 2 (insbesondere Mg, Ca, Sr, Ba), 7 (insbesondere Mn), 8 (insbesondere Fe), 9 (insbesondere Co), 10 (insbesondere Ni), 11 (insbesondere Cu) und 12 (insbesondere Zn) des Periodensystems der Elemente. Beispiele verwendbarer dreiwertiger Metallkationen sind insbesondere die dreiwertigen Kationen von Metallen der Gruppen 3 einschließlich der Lanthaniden (insbesondere Sc, Y, La, Ce), 8 (insbesondere Fe), 11 (insbesondere Au), 13 (insbesondere Al) und 14 (insbesondere Bi) des Periodensystems der Elemente. Beispiele verwendbarer vierwertiger Kationen sind insbesondere die vierwertigen Kationen von Metallen der Lanthaniden (insbesondere Ce) sowie der Gruppe 4 (insbesondere Ti, Zr, Hf) des Periodensystems der Elemente. Die Metallkationen können sowohl allein als auch im Gemisch untereinander eingesetzt werden. Besonders bevorzugt ist die Verwendung dreiwertiger Metallkationen. Ganz besonders bevorzugt ist die Verwendung von Aluminiumkationen.

Von den genannten Metall-Kationen sind alle Metallsalze geeignet, die eine ausreichende Löslichkeit in dem zu verwendenden Lösungsmittel besitzen. Besonders geeignet sind Metallsalze mit schwach komplexierenden Anionen wie zum Beispiel Chlorid, Nitrat und Sulfat, Hydrogensulfat, Carbonat, Hydrogencarbonat, Nitrat, Phosphat, Hydrogenphosphat, oder Dihydrogenphosphat. Bevorzugt sind Salze von Mono- und Dicarbonsäuren, Hydroxisäuren, Ketosäuren sowie Aminosäuren oder basische Salze. Beispielsweise genannt seien bevorzugt Acetate, Propionate, Tartrate, Maleate, Citrate, Laktate, Malate, Succinate. Ebenso bevorzugt ist die Verwendung von Hydroxiden, sofern diese löslich sind. Besonders bevorzugt ist die Verwendung von 2-Hydroxicarbonsäuresalzen wie Citraten und Laktaten. Beispiele besonders bevorzugter Metallsalze sind Alkali- und Erdalkalimetallaluminate und deren Hydrate, etwa Natriumaluminat und dessen Hydrate, Alkali- und Erdalkalimetallacetate und -citrate und deren Hydrate, Aluminiumacetat, Aluminiumpropionat, Aluminiumcitrat und Aluminiumlaktat.

Die genannten Kationen und Salze können in Reinform oder als Gemisch verschiedener Kationen oder Salze verwendet werden. Die eingesetzten Salze des zwei und/oder dreiwertigen Metallkations können weitere Nebenbestandteile wie noch unneutralisierte Carbonsäure und/oder Alkalisalze der neutralisierten Carbonsäure enthalten. Bevorzugte Alkalisalze sind die des Natriums, Kaliums und des Ammoniums. Sie werden typischerweise als wässerige Lösung eingesetzt, welche durch Auflösen der festen Salze in Wasser gewonnen wird, oder bevorzugt direkt als solche erzeugt wird, wodurch gegebenenfalls Trocknungs- und Reinigungsschritte vermieden werden. Vorteilhaft können auch die Hydrate der genannten Salze eingesetzt werden, die sich oft schneller in Wasser lösen als die wasserfreien Salze.

Die Einsatzmenge an Metallsalz beträgt im Allgemeinen mindestens 0,001 Gew.-%, vorzugsweise mindestens 0,01 Gew.-% und in besonders bevorzugter Form mindestens 0,1 Gew.-%, beispielsweise mindestens 0,4 Gew.-% sowie im Allgemeinen höchstens 5 Gew.-%, vorzugsweise höchstens 2,5 Gew.-% und in besonders bevorzugter Form höchstens 1 Gew.-%, beispielsweise höchstens 0,7 Gew.-% jeweils bezogen auf die Masse des Grundpolymeren.

Das Salz des dreiwertigen Metallkations kann als Lösung oder Suspension eingesetzt werden. Als Lösungsmittel für die Metallsalze können Wasser, Alkohole, DMF, DMSO sowie Mischungen dieser Komponenten eingesetzt werden. Besonders bevorzugt sind Wasser und Wasser/Alkohol-Mischungen wie zum Bespiel Wasser/Methanol, Wasser/1,2-Propandiol und Wasser/1,3-Propandiol.

Die Behandlung des Grundpolymeren mit Lösung eines zwei- oder mehrwertigen Kations erfolgt in gleicher Weise wie die mit Oberflächennachvernetzer, einschließlich des Trocknungsschritts. Oberflächennachvernetzer und polyvalentes Kation können in einer gemeinsamen Lösung oder als getrennte Lösungen aufgesprüht werden. Das Aufsprühen der Metallsalz-Lösung auf die Superabsorberpartikel kann sowohl vor als auch nach der Oberflächennachvernetzung erfolgen. In einem besonders bevorzugten Verfahren erfolgt die Aufsprühung der Metallsalz-Lösung im gleichen Schritt mit dem Aufsprühen der Vernetzer-Lösung, wobei beide Lösungen getrennt nacheinander oder gleichzeitig über zwei Düsen aufgesprüht werden, oder Vernetzer- und Metallsalz-Lösung vereint über eine Düse aufgesprüht werden können.

Es können auch alle weiteren, in der Oberflächennachvernetzung von Superabsorbern bekannten Zusätze zugegeben werden. Beispiele sind basische Salze eines zweiwertigen Metallkations wie Calcium oder Strontium, meist als Hydroxide, Hydrogencarbonate, Carbonate, Acetate, Propionate, Citrate, Glukonate, Laktate, Tartrate, Malate, Succinate, Maleate und/oder Fumarate. Weitere Beispiele sind reduzierende Verbindungen wie Hypophosphite, Phosphonsäurederivate, Sulfinate oder Sulfite.

Bevorzugterweise werden neben der Zugabe von röntgenamorphem Aluminiumhydroxid keine weiteren mehrwertigen Metallionen aufgebracht.

Sofern im Anschluss an die Oberflächennachvernetzung und/oder Behandlung mit Komplexbildner ein Trocknungsschritt durchgeführt wird, ist es vorteilhaft, aber nicht unbedingt notwendig, das Produkt nach der Trocknung zu kühlen. Die Kühlung kann kontinuierlich oder diskontinuierlich erfolgen, bequemerweise wird das Produkt dazu kontinuierlich in einen dem Trockner nachgeschalteten Kühler gefördert. Dazu kann jeder zur Abfuhr von Wärme aus pulverförmigen Feststoffen bekannte Apparat verwendet werden, insbesondere jede oben als Trocknungsapparat erwähnte Vorrichtung, sofern sie nicht mit einem Heizmedium, sondern mit einem Kühlmedium wie etwa mit Kühlwasser beaufschlagt wird, so dass über die Wände und je nach Konstruktion auch über die Rührorgane oder sonstige Wärmeaustauschflächen keine Wärme in den Superabsorber eingetragen, sondern daraus abgeführt wird. Bevorzugt ist die Verwendung von Kühlern, in denen das Produkt bewegt wird, also gekühlten Mischern. beispielsweise Schaufelkühlern, Scheibenkühlern oder Paddelkühlern. Der Superabsorber kann auch in der Wirbelschicht durch Einblasen eines gekühlten Gases wie kalter Luft gekühlt werden. Die Bedingungen der Kühlung werden so eingestellt, dass ein Superabsorber mit der für die Weiterverarbeitung gewünschten Temperatur erhalten wird. Typischerweise wird eine mittlere Verweilzeit im Kühler von im Allgemeinen mindestens 1 Minute, vorzugsweise mindestens 3 Minuten und in besonders bevorzugter Form mindestens 5 Minuten sowie im Allgemeinen höchstens 6 Stunden, vorzugsweise höchstens 2 Stunden und in besonders bevorzugter Weise höchstens 1 Stunde eingestellt und die Kühlleistung so bemessen, dass das erhaltene Produkt eine Temperatur von im Allgemeinen mindestens 0 °C, vorzugsweise mindestens 10 °C und in besonders bevorzugter Form mindestens 20 °C sowie im Allgemeinen höchstens 100 °C, vorzugsweise höchstens 80 °C und in besonders bevorzugter Form höchstens 60 °C aufweist.

Der oberflächennachvernetzte Superabsorber wird wahlweise in üblicher Weise gemahlen und/oder gesiebt. Mahlung ist hier typischerweise nicht erforderlich, meist ist aber zur Einstellung der gewünschten Partikelgrößenverteilung des Produkts das Absieben von gebildeten Agglomeraten oder Feinkorn angebracht. Agglomerate und Feinkorn werden entweder verworfen oder vorzugsweise in bekannter Weise und an geeigneter Stelle in das Verfahren zurückgeführt; Agglomerate nach Zerkleinerung. Die für oberflächennachvernetzte Superabsorber gewünschten Partikelgrößen sind die gleichen wie bei Grundpolymeren.

Wird röntgenamorphes Aluminiumhydroxid nach einer Oberflächennachvernetzung zugegeben, kann dies bequemerweise im nachgeschalteten Kühler erfolgen, aber auch in einem eigenen Michsapparat. Das röntgenamorphe Aluminiumhydroixd kann sowohl jeweils vor als auch nach einem auf eine Oberflächennachvernetzung folgenden Mahl- und Siebschritt zugegeben werden.

Wahlweise werden die mit dem erfindungsgemäßen Verfahren hergestellten erfindungsgemäßen Superabsorber mit weiteren Zusätzen versehen, als nicht einschränkende Beispiele solchen, die gegen Verfärbung stabilisieren, die Verbackungsneigung verringern oder die Permeabilität weiter erhöhen. Hierzu können alle bekannten Zusätze auf die für sie jeweils bekannte Weise im erfindungsgemäßen Verfahren eingesetzt werden. Beispiele von bekannten, gegen Verfärbung stabilisierenden Zusätzen sind die oben genannten Sulfonsäure- oder Phosphonsäurederivate, die statt oder zusätzlich zur Zugabe während der Herstellung des erfindungsgemäßen Superabsorbers auch nach seiner Herstellung aufgebracht werden können. Beispiele für bekannte Zusätze, welche die Verbackungsneigung des Superabsorbers verringern oder die Permeabilität weiter erhöhen, sind wasserunlösliche anorganische Pulver.

Meist wird das erfindungsgemäß zugesetzte röntgenamorphe Aluminiumhydroxid auch die Verbackungsneigung des Superabsorbers ausreichend reduzieren. Genügt die erfindungsgemäß zugesetzte Menge dafür nicht, kann sie auch über die oben genannten Grenzen hinaus soweit erhöht werden, dass die erwünschte Reduzierung der Verbackungsneigung erreicht wird. Diese zusätzliche Menge röntgenamorphes Aluminiumhydroxid kann auch in einer zweiten Portion zugesetzt werden wie andere wasserunlösliche anorganische Pulver auch. Typischerweise ist die zusätzliche Menge röntgenamorphes Aluminiumhydroxid auch so groß wie die anderer wasserunlöslicher anorganischer Pulver.

Falls ein weiteres anorganisches Pulver zugesetzt wird, werden bevorzugterweise gefälltes oder durch Pyrolyse hergestelltes Siliciumdioxid sowie durch Pyrolyse hergestelltes Aluminiumoxid verwendet. Pyrogenes Siliciumdioxid ist beispielsweise unter der Marke AEROSIL® und pyrogenes Aluminiumoxid beispielsweise unter der Marke AEROXIDE® Alu von Evonik Industries AG, Inorganic Materials, Rodenbacher Chaussee 4, 63457 Hanau-Wolfgang, Deutschland, erhältlich. Durch Fällung erzeugtes Siliciumdioxid ist beispielsweise unter der Marke SIPER-NAT® von Evonik Industries AG, Inorganic Materials, Rodenbacher Chaussee 4, 63457 Hanau-Wolfgang, Deutschland, erhältlich. Die wasserunlöslichen anorganischen Pulver können durch geeignete Oberflächenbehandlung auch hydrophobiert werden und werden von Herstellern oft sowohl in hydrophobierter als auch in hydrophiler Form angeboten. Im Rahmen dieser Erfindung ist die Verwendung hydrophiler wasserunlöslicher anorganischer Pulver bevorzugt.

Im Allgemeinen wird das wasserunlösliche anorganische Pulver dem Superabsorber in einer Menge von mindestens 0,005 Gew.-%, vorzugsweise von mindestens 0,03 Gew.-% und in besonders bevorzugter Form von mindestens 0,05 Gew.-% sowie im Allgemeinen von höchstens 6,0 Gew.-%, vorzugsweise höchstens 1,0 Gew.-% und in besonders bevorzugter Form höchstens 0,5 Gew.-% zugegeben, jeweils bezogen auf das Gesamtgewicht des wasserfreien Superabsorbers mit anorganischem Pulver.

Die Vermischung von Superabsorbern mit den optionalen Zusätzen kann durch jedes bekannte Mischverfahren erfolgen. Sie werden, sofern in fester Form, in Substanz oder als Suspension in einem Lösungs- oder Suspensionsmittel eingemischt, sofern in gelöster oder flüssiger Form, wahlweise auch in Lösung oder flüssiger Form. Vorzugsweise werden die Zusätze aufgrund der leichteren homogenen Verteilung dem Superabsorber als Pulver oder Suspension eingemischt. Dabei wird nicht notwendigerweise eine einfach durch mechanische Maßnahmen trennbare physikalische Mischung erzeugt. Die Zusatzstoffe können durchaus eine festere Verbindung mit dem Superabsorber eingehen, beispielsweise als vergleichsweise fest anhaftende Oberflächenschicht oder als fest an der Oberfläche der Superabsorberpartikel anhaftende Partikel. Die Einmischung der Zusatzstoffe in den bekannten Superabsorber kann durchaus auch als "Beschichtung" verstanden und bezeichnet werden.

Falls zur Beschichtung eine Lösung oder Suspension verwendet wird, wird als Lösungs- oder Suspensionsmittel ein Lösungs- oder Suspensionsmittel verwendet, das sowohl mit dem Superabsorber wie auch mit dem Zusatzstoff chemisch verträglich ist, also keine unerwünschten chemischen Reaktionen damit eingeht. Typischerweise wird Wasser oder ein organisches Lösungsmittel verwendet, beispielsweise ein Alkohol oder Polyol, oder Mischungen davon. Beispiele geeigneter Lösungs- oder Suspensionsmittel sind Wasser, Isopropanol/Wasser, 1,3-Propandiol/Wasser und Propylenglykol/Wasser, wobei das Mischungsmassenverhältnis vorzugsweise von 20:80 bis 40:60 beträgt. Wird ein Suspensionsmittel für die erfindungsgemäß zu verwendenden Stabilisatoren oder den anorganischen partikulären Feststoff verwendet, so ist Wasser bevorzugt. Der Lösung oder Suspension kann ein Tensid zugesetzt werden.

Optionale Zusatzstoffe werden, sofern sie nicht der Monomermischung oder dem polymerisierenden Gel zugegeben werden, im Allgemeinen auf genau die gleiche Weise mit dem Superabsorber vermischt wie die zur Oberflächennachvernetzung auf den Superabsorber aufgebrachte, einen Oberflächennachvernetzer enthaltende Lösung oder Suspension. Der Zusatzstoff kann als Bestandteil der zur Oberflächennachvernetzung aufgebrachten Lösung oder einer ihrer Komponenten auf einen (noch) nicht nachvernetzten Superabsorber (ein "Grundpolymer" oder "Basispolymer") aufgebracht, also der Lösung des Oberflächennachvernetzers oder einer ihrer Komponenten zugesetzt werden. Der mit Oberflächennachvernetzungsmittel und Zusatzstoffen beschichtete Superabsorber durchläuft dann die weiteren zur Oberflächennachvernetzung erforderlichen Verfahrensschritte, beispielsweise eine thermisch induzierte Reaktion des Oberflächennachvernetzungsmittels mit dem Superabsorber. Dieses Verfahren ist vergleichsweise einfach und wirtschaftlich.

Falls der Superabsorber nach der Oberflächennachvernetzung oder der Komplexierung einem Kühlschritt unterworfen wird, können die wahlweisen Zusätze bequemerweise im Kühler untergemischt werden. Falls Zusatzstoffe als Lösung oder Suspension aufgebracht werden, kann die Aufbringung auch auf den bereits oberflächennachvernetzten Superabsorber in den gleichen Mischapparaten wie für die Aufbringung des Oberflächennachvernetzungsmittels auf das Grundpolymer beschrieben erfolgen. Meist, aber nicht notwendigerweise wird anschließend ebenso wie bei der Oberflächennachvernetzung erwärmt, um den Superabsorber wieder zu trocknen. Die bei dieser Trocknung eingestellte Temperatur liegt dann aber im Allgemeinen bei höchstens 110 °C, vorzugsweise höchstens 100 °C und in besonders bevorzugter Weise höchstens 90 °C, um unerwünschte Reaktionen des Zusatzstoffs zu vermeiden. Die Temperatur wird so eingestellt, dass in Anbetracht der Verweilzeit im Trocknungsaggregat der gewünschte Wassergehalt des Superabsorbers erreicht wird. Es ist durchaus auch möglich und bequem, Zusatzstoffe einzeln oder gemeinsam mit anderen üblichen Hilfsmitteln, beispielsweise Staubbindemitteln, Mitteln gegen Verbackung oder Wasser zur Rückbefeuchtung des Superabsorbers zuzugeben. Die Temperatur der Polymerpartikel beträgt in diesem Fall zwischen 0 °C und 190 °C, bevorzugt weniger als 160 °C, mehr bevorzugt weniger als 130 °C, noch mehr bevorzugt weniger als 100 °C, und am meisten bevorzugt weniger als 70 °C. Die Polymerpartikel werden gegebenenfalls nach Beschichtung zügig auf Temperaturen unterhalb einer etwaigen Zersetzungstemperatur des Zusatzstoffs abgekühlt.

Wahlweise können auf die Oberfläche der Superabsorberpartikel, ob nicht nachvernetzt oder nachvernetzt, im Herstellverfahren in jedem Prozessschritt bei Bedarf alle bekannten Beschichtungen, wie filmbildende Polymere, thermoplastische Polymere, Dendrimere, polykationische Polymere (wie beispielsweise Polyvinylamin, Polyethylenimin oder Polyallylamin), oder alle dem Fachmann bekannten wasserlöslichen mono- oder polyvalenten Metallsalze, wie beispielsweise Aluminiumsulfat, Natrium-, Kalium-, Zirkonium- oder Eisensalze zusätzlich aufgebracht werden. Beispiele für nützliche Alkalimetallsalze sind Natrium- und Kaliumsulfat, Natrium- und Kaliumlactate, -citrate, -sorbate. Dadurch können zusätzliche Effekte, beispielsweise eine verringerte Verbackungsneigung des Endprodukts oder des Zwischenprodukts im jeweiligen Prozessschritt des Herstellverfahrens, verbesserte Verarbeitungseigenschaften oder eine weiter gesteigerte Permeabilität (SFC) erreicht werden. Wenn Zusatzstoffe in Form von Dispersionen eingesetzt und aufgesprüht werden, dann werden sie bevorzugt als wässrige Dispersionen eingesetzt, und es wird bevorzugt noch zusätzlich ein Entstaubungsmittel zur Fixierung des Additivs auf der Oberfläche des Superabsorbers aufgebracht. Das Entstaubungsmittel wird dann entweder direkt der Dispersion des anorganischen pulvrigen Additivs hinzugefügt, optional kann es auch als separate Lösung vor, während, oder nach dem Auftrag des anorganischen pulvrigen Additivs durch Aufsprühen hinzugefügt werden. Am meisten bevorzugt ist das gleichzeitige Aufsprühen von Nachvernetzungsmittel, Entstaubungsmittel und pulvrigem anorganischen Additiv in der Nachvernetzung. In einer weiteren bevorzugten Verfahrensvariante wird das Entstaubungsmittel aber separat im Kühler zugegeben, beispielsweise durch Aufsprühen von oben, unten oder von der Seite. Besonders geeignete Entstaubungsmittel, die auch zur Fixierung pulvriger anorganischer Additive an der Oberfläche der wasserabsorbierenden Polymerpartikel dienen können, sind Polyethylenglykole mit einem Molekulargewicht von 400 bis 20000 g/mol, Polyglyzerin, 3- bis 100-fach ethoxylierte Polyole, wie Trimethylolpropan, Glyzerin, Sorbitol und Neopentylglykol. Besonders geeignet sind 7- bis 20-fach ethoxyliertes Glyzerin oder Trimethylolpropan, wie beispielsweise Polyol TP 70® (Perstorp, SE). Letztere haben insbesondere den Vorteil, dass sie die Oberflächenspannung eines wässrigen Extrakts der wasserabsorbierenden Polymerpartikel nur unwesentlich herabsetzen.

Es ist ebenso möglich, den erfindungsgemäßen Superabsorber durch Wasserzusatz auf einen gewünschten Wassergehalt einzustellen. Es kann auch vorteilhaft sein, den Superabsorber durch Wasserzugabe anzuquellen und danach wieder durch Trocknung auf den gewünschten Wassergehalt einzustellen.

Alle Beschichtungen, Feststoffe, Zusätze und Hilfsstoffe können jeweils in separaten Verfahrensschritten zugegeben werden, meist ist jedoch die bequemste Methode, sie - falls sie nicht während der Versetzung des Grundpolymers mit Oberflächennachvernetzungsmittel zugegeben werden - dem Superabsorber im Kühler zuzugeben, etwa durch Aufsprühen einer Lösung oder Zugabe in feinteiliger fester oder in flüssiger Form.

Die erfindungsgemäßen Superabsorber weisen im Allgemeinen eine Zentrifugenretentionskapazität (CRC, Messmethode s. unten) von mindestens 5 g/g, vorzugsweise von mindestens 10 g/g und in besonders bevorzugter Form von mindestens 20 g/g auf. Üblicherweise liegt sie für oberflächennachvernetzte Superabsorber nicht über 40 g/g, für Grundpolymere liegt sie allerdings oft höher.

Die erfindungsgemäßen Superabsorber weisen, sofern sie oberflächennachvernetzt sind, typischerweise eine Absorption unter Druck (AUL0.9psi, Messmethode s. unten) von mindestens 10 g/g, vorzugsweise mindestens 14 g/g, bevorzugt mindestens 18 g/g und ganz besonders bevorzugt mindestens 22 g/g und üblicherweise nicht über 30 g/g auf.

Ein weiterer Gegenstand der vorliegenden Erfindung sind Hygieneartikel, enthaltend erfindungsgemäße Superabsorber, vorzugsweise ultradünne Windeln, enthaltend eine absorbierende Schicht bestehend aus 50 bis 100 Gew.-%, vorzugsweise 60 bis 100 Gew.-%, bevorzugt 70 bis 100 Gew.-%, besonders bevorzugt 80 bis 100 Gew.-%, ganz besonders bevorzugt 90 bis 100 Gew.-%, erfindungsgemäßer Superabsorber, wobei die Umhüllung der absorbierenden Schicht selbstverständlich nicht berücksichtigt ist.

Ganz besonders vorteilhaft sind die erfindungsgemäßen Superabsorber auch zur Herstellung von Laminaten und Kompositstrukturen, wie sie beispielsweise in der US 2003/0181115 sowie der US 2004/0019342 beschrieben sind, geeignet. Zusätzlich zu den in beiden Schriften zur Herstellung solcher neuer absorbierenden Strukturen beschriebenen Schmelzklebern und insbesondere den in der US 2003/0181115 beschriebenen Fasern aus Schmelzklebern, an die die Superabsorberpartikel gebunden ist, eignen sich die erfindungsgemäßen Superabsorber auch zur Herstellung von vollkommen analogen Strukturen unter Verwendung von UV-vernetzbaren Schmelzklebern, welche beispielsweise als AC-Resin® (BASF SE, Ludwigshafen, Deutschland) vertrieben werden. Diese UV-vernetzbaren Schmelzkleber haben den Vorteil bereits bei 120 bis 140 °C verarbeitbar zu sein, daher sind sie mit vielen thermoplastischen Substraten besser kompatibel. Ein weiterer wesentlicher Vorteil besteht darin, dass UV-vernetzbare Schmelzkleber toxikologisch sehr unbedenklich sind und auch keine Ausdünstungen in den Hygieneartikeln verursachen. Ein ganz wesentlicher Vorteil, im Zusammenhang mit den erfindungsgemäßen Superabsorbern, ist die Eigenschaft der UV-vernetzbaren Schmelzkleber während der Verarbeitung und Vernetzung nicht zur Vergilbung zu neigen. Dies ist insbesondere von Vorteil, wenn ultradünne oder teilweise transparente Hygieneartikel hergestellt werden sollen. Die Kombination der erfindungsgemäßen Superabsorber mit UV-vernetzbaren Schmelzklebern ist daher besonders vorteilhaft. Geeignete UV-vernetzbare Schmelzkleber sind beispielsweise beschrieben in EP 0 377 199 A2, EP 0 445 641 A1, US 5,026,806, EP 0 655 465 A1 und EP 0 377 191 A2.

Der erfindungsgemäße Superabsorber kann außerdem in anderen Gebieten der Technik eingesetzt werden, bei denen Flüssigkeiten, insbesondere Wasser oder wässrige Lösungen absorbiert werden. Diese Gebiete sind beispielsweise Lagerung, Verpackung, Transport (als Bestandteile von Verpackungsmaterial für wasser- oder feuchtigkeitsempfindliche Artikel, etwa zum Blumentransport, auch als Schutz gegen mechanische Einwirkungen); Tierhygiene (in Katzenstreu); Lebensmittelverpackung (Transport von Fisch, Frischfleisch; Absorption von Wasser, Blut in Frischfisch-oder-fleischverpackungen); Medizin (Wundpflaster, wasserabsorbierendes Material für Brandverbände oder für andere nässende Wunden), Kosmetik (Trägermaterial für Pharmachemikalien und Medikamente, Rheumapflaster, Ultraschallgel, Kühlgel, Kosmetikverdicker, Sonnenschutz); Verdicker für Öl/Wasser bzw. Wasser/Öl-Emulsionen; Textilien (Feuchtigkeitsregulation in Textilien, Schuheinlagen, zur Verdampfungskühlung, etwa in Schutzkleidung, Handschuhen, Stirnbändern); chemisch-technische Anwendungen (als Katalysator für org. Reaktionen, zur Immobilisierung großer funktioneller Moleküle wie Enzymen, als Adhäsionsmittel bei Agglomerationen, Wärmespeicher, Filtrationshilfsmittel, hydrophile Komponente in Polymerlaminaten, Dispergiermittel, Verflüssiger); als Hilfsmittel beim Pulverspritzguss, im Bau- und Konstruktionswesen (Installation, in lehmbasierenden Putzen, als vibrationshemmendes Medium, Hilfsmittel bei Tunnelgrabungen in wasserreichem Untergrund, Kabelummantelung); Wasserbehandlung, Abfallbehandlung, Wasserabtrennung (Enteisungsmittel, wiederverwendbare Sandsäcke); Reinigung; Agrarindustrie (Bewässerung, Rückhaltung von Schmelzwasser und Tauniederschläge, Kompostierungszusatz, Schutz der Wälder vor Pilz-/Insektenbefall, verzögerte Freisetzung von Wirkstoffen an Pflanzen); zur Brandbekämpfung oder zum Brandschutz; Coextrusionsmittel in thermoplastischen Polymeren (z. B. zur Hydrophilierung von Mehrschichtfolien); Herstellung von Folien und thermoplastischen Formkörpern, die Wasser absorbieren können (z.B. Regen- und Tauwasser speichernde Folien für die Landwirtschaft; Superabsorber enthaltende Folien zum Frischhalten von Obst und Gemüse, die in feuchte Folien verpackt werden; Superabsorber-Polystyrol Coextrudate, beispielsweise für Lebensmittelverpackungen wie Fleisch, Fisch, Geflügel, Obst und Gemüse); oder als Trägersubstanz in Wirkstoffformulierungen (Pharma, Pflanzenschutz).

Die erfindungsgemäßen Artikel zur Absorption von Flüssigkeit unterscheiden sich von bekannten dadurch, dass sie den erfindungsgemäßen Superabsorber enthalten.

Es wurde außerdem ein Verfahren zur Herstellung von Artikeln zur Absorption von Flüssigkeit, insbesondere Hygieneartikeln gefunden, das dadurch gekennzeichnet ist, dass man bei der Herstellung des betreffenden Artikels mindestens einen erfindungsgemäßen Superabsorber einsetzt. Im Übrigen sind Verfahren zur Herstellung solcher Artikel unter Einsatz von Superabsorber bekannt.

Testmethoden

Der Superabsorber wird mit den nachfolgend beschriebenen Testmethoden geprüft.

Die nachfolgend beschriebenen, mit "NWSP" bezeichneten Standard-Testmethoden werden beschrieben in: "Nonwovens Standards Procedures", Ausgabe 2015, gemeinsam herausgegeben von EDANA (European Disposables and Nonwovens Association, Avenue Herrmann Debroux 46, 1160 Brüssel, Belgien, www.edana.org) und INDA (Association of the Nonwoven Fabrics Industry, 1100 Crescent Green, Suite 115, Cary, North Carolina 27518, U.S.A., www.inda.org). Diese Veröffentlichung ist sowohl von EDANA als auch von INDA erhältlich.

Alle nachfolgend beschriebenen Messungen sollten, wenn nicht anders angegeben, bei einer Umgebungstemperatur von 23 ± 2 °C und einer relativen Luftfeuchte von 50 ± 10 % durchgeführt werden. Die Superabsorberpartikel werden vor der Messung gut durchmischt, wenn nicht anders angegeben.

Zentrifugenretentionskapazität (CRC, Centrifuge Retention Capacity)

Die Zentrifugenretentionskapazität des Superabsorbers wird gemäß der in US 2007/0 135 785 A1, Absätze [0153] bis [0157] beschriebenen Methode bestimmt.

Absorption unter Druck (AUL0.9psi, "Absorbency Under Load of 0.9 psi")

Die Absorption unter einem Druck von 6205 Pa (0.9 psi) des Superabsorbers wird gemäß der in US 2014/0 306 156 A1, Absätze [0124] bis [0143] beschriebenen Methode bestimmt.

Absorption unter Druck (AUL0.3psi, "Absorbency Under Load of 0.3 psi")

Die Absorption unter einem Druck von 2068 Pa (0.3 psi) des Superabsorbers wird gemäß der Standard-Testmethode Nr. NWSP 242.0 R2 (15) "Gravimetric Determination of Absorption Against Pressure" bestimmt, jedoch mit einem Gewicht (s. Ziff. 6.5 der Methodenbeschreibung), mit dem ein Druck von 2068 Pa statt 4826 Pa (entspricht 21 g/cm2 statt 49 g/cm2) eingestellt wird.

Volumetrische Absorption unter Druck (VAUL, "Volumetric Absorbency under Load")

Die volumetrische Absorption unter Druck des Superabsorbers wird gemäß der in US 2015/0 299 404 A1, Absätze [0386] bis [0398] beschriebenen Methode bestimmt. In Tabelle 1 wird der bei einem Druck von 2068 Pa (0.3 psi) ermittelte $\tau$-Wert angegeben.

Feuchtegehalt des Superabsorbers (Restfeuchte, Wassergehalt)

Der Wassergehalt des Superabsorbers wird gemäß der Standard-Testmethode Nr. NWSP 230.0 R2 (15) "Estimation of the Moisture Content as Weight Loss Upon Heating" bestimmt.

Partikelgrößenverteilung

Die Partikelgrößenverteilung des Superabsorbers wird gemäß der Standard-Testmethode Nr. NWSP 220.0 R2 (15) "Determination of Polyacrylate Superabsorbent Powders and Particle Size Distribution - Sieve Fractionation" bestimmt.

Extrahierbare Anteile

Die extrahierbaren Anteile des Superabsorbers werden gemäß der Standard-Testmethode Nr. NWSP 270.0 R2 (15) "Determination of Extractable Polymer Content by Potentiometric Titration" bestimmt.

Permeabilität (SFC, "Saline Flow Conductivity")

Die Permeabilität einer vom Superabsorber durch Flüssigkeitsabsorption gebildeten gequollenen Gelschicht wird unter Druckbelastung von 0.3 psi (2068 Pa), wie in EP 0 640 330 A1 beschrieben, als Gel-Layer-Permeability einer gequollenen Gelschicht aus Superabsorberpartikeln bestimmt, wobei die in zuvor genannter Patentanmeldung auf Seite 19 und in Figur 8 beschriebene Apparatur dahingehend modifiziert ist, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Permeabilität (SFC) wird wie folgt berechnet:

$$SFC\ [cm^3s/g] = (Fg(t=0)xL0)/(dxAxWP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in g/cm3, A die Fläche der Gelschicht in cm2 und WP der hydrostatische Druck über der Gelschicht in dyn/cm2 darstellt.

Permeabilität (GBP, "Gel Bed Permeability")

Die Gelbettpermeabilität wird nach der Vorschrift in der veröffentlichten Patentanmeldung Nr. US 2005/0 256 757 A1, Absätze [0061] bis [0075] gemessen.

Beispiele

Das in den folgenden Beispielen verwendete Grundpolymer wurde durch Polymerisation einer wässrigen Monomerlösung hergestellt, die Natriumacrylat und Acrylsäure (entsprechend einem Neutralisationsgrad der Acrylsäure von 71 Mol-%) in einer Konzentration von 41 Gew.% (Natriumacrylat plus Acrylsäure bezogen auf die Gesamtmenge), weiterhin 0,75 Gew.% (bezogen auf unneutralisierte Acrylsäure) Polyethylenglykol-4000 (Polyethylenglykol mit einem gewichtsmittleren Molgewicht von 4000 g/Mol) und 0,46 Gew.-% (bezogen auf unneutralisierte Acrylsäure) Triacrylsäureester dreifach ethoxylierten Glycerins enthielt. Als Initiatorsystem wurden (jeweils auf unneutralisierte Acrylsäure bezogen) 0,184 Gew.-% Natriumpersulfat, 0,0007 Gew.- Wasserstoffperoxid und 0,0026 Gew.-% Ascorbinsäure verwendet. Die Polymerisation erfolgte in einem Kneter. Zur besseren Trocknung wurde das erhaltene Gel extrudiert und anschließend getrocknet, gemahlen und daraus der Siebschnitt von 150 bis 710 $\mu$m gewonnen. Das so hergestellte Grundpolymer hatte eine CRC von 36,5 g/g, eine AUL 0.3 psi von 14.6 g/g und enthielt extrahierbare Anteile von 13,0 Gew.-%. Die mittels Siebanalyse erhaltene Partikelgrößenverteilung war:

| | |
|---|---|
| > 850 $\mu$m | < 0,1 Gew.-% |
| 600 - 850 $\mu$m | 10,6 Gew.-% |
| 300 - 600 $\mu$m | 70,8 Gew.-% |
| 100 - 300 $\mu$m | 18,0 Gew.-% |
| < 100 $\mu$m | < 0,5 Gew.-% |

Derartige Grundpolymere sind gängig und auch kommerziell erhältlich, beispielsweise von BASF SE, Ludwigshafen, Deutschland.

Der in den Beispielen verwendete Mischer war ein Pflugschar®-Mischer mit 5 l Volumen, Typ VT 5R-MK, mit Heizmantel von Gebr. Lödige Maschinenbau GmbH; Elsener Straße 7-9, 33102 Paderborn, Deutschland. Zur Messung der Temperatur des Produkts im Mischer wurde ein Thermoelement in die am Mischer dafür vorgesehene Öffnung so weit eingeführt, dass seine Spitze zwar von der beheizten Innenwand des Mischers beabstandet war und sich im Produkt befand, aber nicht von den Mischwerkzeugen erfasst werden konnte. Zur Zugabe von Aluminiumhydroxid in Beispielen 1-6 wurde ein gleicher Mischer, jedoch ohne Heizmantel und Thermoelement verwendet.

Das in den Beispielen verwendete röntgenamorphe Aluminiumhydroxid war Aluminiumhydroxid Trockengel, Artikel Nr. 511066100, Chargennummer 3048632 von Dr. Paul Lohmann GmbH KG, Hauptstraße 2, 31860 Emmerthal, Deutschland. Im Rasterlektronenmikroskop zeigt sich das Pulver als kugelige Partikel mit Durchmessern im Bereich von 20 - 25 $\mu$m, aber auch einigen kleineren Kugeln im Bereich von 5-10 $\mu$m. Im Röntgendiffraktogramm (gemessen mit einem Diffraktometer D8 Advance Serie 2 von Bruker Corporation, 40 Manning Road, Billerica, MA 01821, U.S.A., mit Mehrfachprobenwechlser, Cu-Anolde, Divergenzblende 0,1° mit ASS und Lynx-Eye, 3 ° Öffnung) wurden keine Beugungslinien gemessen, was auf eine Größe der Primärkristallite von deutlich kleiner als 2 nm deutet.

Das in den Vergleichsbeispielen verwendete kristalline Aluminumhydroxid war Hydrargillit Emplura®, Bestellnr. 1010911000 von Merck KGaA, Frankfurter Straße 250, 64293 Darmstadt. Im Rasterlektronenmikroskop zeigt sich das Pulver als unregelmäßige, vorwiegend plättchenförmige Partikel mit Dimensionen im Bereich von 5-50 $\mu$m. Im Röntgendiffraktogramm wurden die für Hydrargillit erwarteten Beugungslinien gemessen, bei einer Größe der Primärkristallite von mehr als 200 nm.

Beispiel 1

1,2 kg Superabsorber-Grundpolymer wurden im Mischer vorgelegt. Bei 23 °C und einer Wellendrehzahl von 200 Umdrehungen pro Minute wurde mittels einer mit Stickstoff betriebenen Zweistoff-Sprühdüse eine Lösung aus 0,08 Gew.-% Ethylenglykoldiglycidylether, 2,5 Gew.-% 1,2-Propandiol und 3 Gew.-% Wasser, jeweils auf das Grundpolymer bezogen, aufgesprüht. Anschließend wurde die Wellendrehzahl auf 60 Umdrehungen pro Minute reduziert, die Produkttemperatur auf 130 °C erhöht und anschließend für 30 Minuten gehalten.

Der erhaltene Superabsorber wurde aus dem Mischer entnommen und Proben analysiert. Die Werte sind in Tabelle 1 angegeben.

Der erhaltene Superabsorber wurde direkt danach (die Produkttemperatur betrug dann etwa 100 °C) in einem weiteren Mischer bei einer Wellendrehzahl von 200 Umdrehungen pro Minute mit 0,5 Gew.-%, bezogen auf den Superabsorber, röntgenamorphem Aluminiumhydroxid vermischt (Mischdauer ca. eine Minute) und der Siebschnitt von 150 - 710 $\mu$m gewonnen.

Der so erhaltene Superabsorber wurde wiederum analysiert, die erhaltenen Messwerte sind in Tabelle 1 angegeben

Beispiel 2

Beispiel 1 wurde wiederholt, wobei jedoch nach Einmischen des Aluminiumhydroxids und vor dem Absieben mittels einer mit Stickstoff betriebenen Zweistoffdüse noch 3,0 Gew.-%, bezogen auf den Superabsorber, Wasser aufgesprüht wurde. Die erhaltenen Messwerte sind in Tabelle 1 angegeben.

Beispiel 3

Beispiel 2 wurde wiederholt, wobei jedoch 0,35 Gew.-% röntgenamorphes Aluminiumhydroxid eingesetzt wurden. Die erhaltenen Messwerte sind in Tabelle 1 angegeben.

Beispiel 4

Beispiel 2 wurde wiederholt, wobei jedoch 0,5 Gew.-% röntgenamorphes Aluminiumhydroxid eingesetzt wurden. Die erhaltenen Messwerte sind in Tabelle 1 angegeben.

Beispiel 5

Beispiel 2 wurde wiederholt, wobei jedoch 0,75 Gew.-% röntgenamorphes Aluminiumhydroxid eingesetzt wurden. Die erhaltenen Messwerte sind in Tabelle 1 angegeben.

Beispiel 6 (Vergleich)

Beispiel 1 wurde wiederholt, wobei jedoch 0,5 Gew.-% kristallines Aluminiumhydroxid statt röntgenamorphem eingesetzt wurden. Die erhaltenen Messwerte sind in Tabelle 1 angegeben.

Auswertung

Die Beispiele 1 bis 6 zeigen, dass durch Aufbringung von Aluminiumhydroxid die GBP gesteigert werden kann, ohne signifikant CRC und AUL zu opfern. Der Vergleich zwischen Beispielen 1 und 6 zeigt, dass die Steigerung der GBP bei röntgenamorphem Aluminiumhydroxid ganz erheblich höher ist als bei kristallinem Aluminiumhydroxid. Der Vergleich zwischen Beispielen 1 und 4 zeigt, dass durch Zugabe von Wasser nach der Zugabe von röntgenamorphem Aluminiumhydroxid eine weitere Steigerung der GBP möglich ist. Die Beispiele 2 bis 5 zeigen, dass unter den hier angewendeten Versuchsbedingungen ein Optimum bei der Zugabe von 0,5 Gew.-% röntgenamorphem Aluminiumhydroxid liegt. Diese Beispiele zeigen weiterhin, dass die Zugabe von röntgenamorphem Aluminiumhydroxid keine signifikanten Auswirkungen auf die Quellkinetik des Superabsorbers hat.

Beispiel 7

1,2 kg Superabsorber-Grundpolymer wurden im Mischer vorgelegt. Bei 23 °C und einer Wellendrehzahl von 200 Umdrehungen pro Minute wurden 0,5 Gew.-%, bezogen auf den Superabsorber, röntgenamorphes Aluminiumhydroxid zugegeben und für 5 Minuten eingemischt. Anschließend wurde bei unveränderter Drehzahl und Temperatur mittels einer mit Stickstoff betriebenen Zweistoff-Sprühdüse eine Lösung aus 0,08 Gew.-% Ethylenglykoldiglycidylether, 2,5 Gew.-% 1,2-Propandiol und 3 Gew.-% Wasser, jeweils auf das Grundpolymer bezogen, aufgesprüht. Anschließend wurde die Wellendrehzahl auf 60 Umdrehungen pro Minute reduziert, die Produkttemperatur auf 130 °C erhöht und anschließend für 30 Minuten gehalten.
Der Superabsorber wurde auf Raumtemperatur abgekühlt und der Siebschnitt von 150 - 710 μm gewonnen. Der erhaltene Superabsorber wurde analysiert, die erhaltenen Messwerte sind in Tabelle 1 angegeben.

Beispiel 8

Beispiel 7 wurde wiederholt, wobei jedoch 0,5 Gew.-% kristallines Aluminiumhydroxid statt röntgenamorphem eingesetzt wurden. Die erhaltenen Messwerte sind in Tabelle 1 angegeben.

Auswertung

Der Vergleich zwischen Beispielen 7 und 8 zeigt wiederum, dass mit röntgenamorphem Aluminiumhydroxid eine erheblich größere Steigerung der GBP erzielt werden kann als mit kristallinem. Der Vergleich zwischen Beispielen 1 und 7 zeigt, dass die Zugabe des Aluminiumhydroxids vor der Oberflächennachvernetzungsreaktion die GBP stärker erhöht als die Zugabe danach. Auch diese Beispiele zeigen, dass die Zugabe von röntgenamorphem Aluminiumhydroxid keine signifikanten Auswirkungen auf die Quellkinetik des Superabsorbers hat.

Beispiel 9 (Vergleich)

Die in EP 233 067 A2, WO 2014/167036 A1, WO 2014/167040 A1 und WO 2014/168858 A1 allgemein beschriebene Verwendung von frisch gefälltem Aluminiumhydroxidsol - also röntgenamorphem, aber nicht pulverförmigem Aluminiumhydroxid - zur Oberflächennachvernetzung von Superabsorbern sollte nachgestellt werden. Bei der Herstellung von Aluminiumhydroxidsol durch Nacharbeitung der in diesen Schriften konkret genannten Vorschriften traten jedoch Schwierigkeiten auf.

EP 233 067 A2 beschreibt (Seiten 14-15) die Bildung von Aluminumhydroxidsol aus 8 Gewichtsteilen Aluminiumchlorid-Hexahydrat und 8 Gewichtsteilen Natriumaluminat in wässriger Lösung. Aufgrund der Molgewichte von Aluminiumchlorid-Hexahydrat von 241 g/Mol und von Natriumaluminat mit 118 g/Mol entspricht dieses Gewichtsverhältnis von 1:1 einem Molverhältnis von 1:2. Nach der Stöchiometrie $AlCl_3$ + 3 $NaAl(OH)_4$ -> 4 $Al(OH)_3$ + 3 NaCl würde die Bildung von Aluminiumhydroxidsol aber das Molverhältnis von 1:3 erfordern. Hier muss also noch relativ saurer pH und deshalb kein $Al(OH)_3$ vorliegen. EP 233 067 A2 nennt dort weiterhin die Bildung von Aluminiumhydroxidsol aus 32 Gewichtsteilen Aluminiumchlorid-Hexahydrat und 15,9 Gewichtsteilen Natriumhydroxid in wässriger Lösung. Aus den Molgewichten (NaOH: 40 g/Mol) errechnet sich das nach der Stöchiometrie $AlCl_3$ + 3 NaOH -> $Al(OH)_3$ + 3 NaCl erforderliche Molverhältnis von 1:3, so dass danach Aluminiumhydroxid entstehen könnte. Nach der Lehre von EP 233 067 A2 wird jedoch zur Herstellung der Oberflächennachvernetzerlösung unmittelbar nach dem Mischen der Reaktanden oder bereits in deren wässrige Lösungen auch Polyol zugegeben, das in den konkreten Herstellvorschriften aber nicht konkret genannt wird.. Damit liegt dort aber auch bei Einsatz der notwendigen Reaktanden in korrekter Stöchiometrie kein $Al(OH)_3$-Sol vor, sondern ein durch Chelatbildung mit dem Polyol stabilisiertes, nicht näher definiertes Al-Salz in Lösung.

Die drei letztgenannten Schriften enthalten gleichlautende Vorschriften zur Herstellung eines "Neutralized Aluminum Salt C" aus 200 g 20 Gew.-%iger wässriger Aluminiumsalzlösung, zu der unter Rühren 130 g 50 Gew.-%ige wässrige Natriumhydroxidlösung zugegeben wird, bis ein pH von 7 erreicht wird. Die so entstehende weiße kolloidale Suspension wäre mit einem Turnax [sic]-Rührer (gemeint ist vermutlich ein Ultra-Turrax® von IKA®-Werke GmbH & Co. KG, Janke & Kunkel-Str. 10, 79219 Staufen, Deutschland) zu homogenisieren und würde danach ohne weitere Reinigung zur Oberflächennachvernetzung eingesetzt. Nach dieser Vorschrift wird Natronlauge weit überdosiert. Für die eingesetzten 200 g Aluminiumsulfatlösung * 20 Gew.-% = 40 g $Al_2(SO_4)_3$ mit Molgewicht 342 g/Mol, also 117 mMol $Al_2(SO_4)_3$ sind nach der Stöchiometrie $Al_2(SO_4)_3$ + 6 NaOH -> 2 $Al(OH)_3$ + 3 $Na_2SO_4$ insgesamt 6 * 117 mMol = 0,7 Mol NaOH zur Bildung einer neutralen Suspension von $Al(OH)_3$ erforderlich. Tatsächlich wurden 130 g NaOH-Lösung * 50 Gew.-% = 65 g NaOH mit Molgewicht 40 g/Mol, also 1,63 Mol eingesetzt, mehr als das Doppelte des stöchiometrisch Notwendigen. Hier kann demnach kein $Al(OH)_3$-Sol erzeugt werden, sondern Natriumaluminatlösung, die basisch sein muss. Der angegebene pH von 7 ist nicht mit der angegebenen Natronlaugemenge vereinbar.

Die drei letztgenannten Schriften enthalten ferner gleichlautende Vorschriften zur Herstellung eines "Neutralized Aluminum Salt D" aus 120 g 20 Gew.-%iger wässriger Aluminiumsalzlösung, zu der unter Rühren 60 g 20 Gew.-%ige wässrige Natriumaluminatlösung zugegeben wird, bis ein pH von 6,5 erreicht wird. Für die eingesetzten 120 g Aluminiumsulfatlösung * 20 Gew.-% = 24 g $Al_2(SO_4)_3$ mit Molgewicht 342 g/Mol, also 70 mMol $Al_2(SO_4)_3$ sind nach der Stöchiometrie $Al_2(SO_4)_3$ + 6 $NaAl(OH)_4$ -> 8 $Al(OH)_3$ + 3 $Na_2SO_4$ insgesamt 8 * 70 mMol = 0,56 Mol Natriumaluminat zur Bildung einer neutralen Suspension von $Al(OH)_3$ erforderlich. Tatsächlich wurden 60 g $NaAl(OH)_4$-Lösung * 20 Gew.-% = 12 g $NaAl(OH)_4$ mit Molgewicht 118 g/Mol, also 102 mMol eingesetzt, also weniger als ein Fünftel des stöchiometrisch Notwendigen. Auch hier wird demnach kein $Al(OH)_3$-Sol erzeugt, sondern im Wesentlichen bleibt Aluminiumsulfatlösung erhalten.

Beispiel 10 (Vergleich)

In einem 450 mL-Becherglas wurden 149,2 g 26,8 Gew.-%ige wässrige Aluminiumsulfatlösung (enthaltend 117 mMol $Al_2(SO_4)_3$) vorgelegt und mit einem Rührfisch magnetisch gerührt. pH-Wert und Temperatur des Inhalts des Becherglases wurden mittels pH-Elektrode und Thermometer gemessen. 59,3 g 50 Gew.-%ige Natronlauge (enthaltend 741 mMol NaOH) wurden in einen Tropftrichter über dem Becherglas gefüllt. Die Natronlauge wurde (1 Tropfen/Sekunde) in das Becherglas getropft. In einer stark exothermen Reaktion entstand gelartiges farbloses Produkt, dass sich zunächst an der pH-Elektrode festsetzte und schließlich vollständig erstarrte. Der gemessene pH-Wert betrug 12,3, wobei die Messung aufgrund des an der Elektrode angesetzten Produkts verfälscht sein kann.

Eine Wiederholung des Versuchs, bei der das Becherglas zur Abfuhr der Reaktionswärme in einem Eisbad platziert und die pH-Elektrode jeweils nur nach der Zugabe von 30 Tropfen Natronlauge zur Messung in die Reaktionsmischung gehalten und danach gereinigt wurde, ergab kein anderes Ergebnis. Der pH stieg auf 5,2, danach wurde der Inhalt des Becherglases fest. Bei einer Wiederholung dieses letztgenannten Versuchs ohne Eisbad wurde der Inhalt des Becherglases bei pH 4,4 fest.

Eine Suspension, die durch Versprühen auf Superabsorber aufgebracht werden könnte, wurde nicht erhalten.

Beispiel 11 (Vergleich)

In einem 200 mL-Becherglas wurden 104 g Wasser vorgelegt und magnetisch gerührt. Das Wasser wurde auf 40 °C erwärmt, daraufhin wurden 26 g Natriumaluminatpulver (220 mMol) zugegeben. Es wurde bei 60 bis 70 °C weiter gerührt, um eine klare 20 Gew.-%ige Lösung zu erhalten, die anschließend auf Raumtemperatur abkühlen gelassen wurde.

In einem 450 mL-Becherglas wurden 89,55 g 26,8 Gew.-%ige wässrige $Al_2(SO_4)_3$-Lösung (entsprechend 24 g $Al_2(SO_4)_3$, 70 mMol) vorgelegt und mit einem Rührfisch magnetisch ge-rührt. pH-Wert und Temperatur des Inhalts des Becherglases wurden mittels pH-Elektrode und Thermometer gemessen. Die Natriumaluminatlösung wurde in einen Tropftrichter über dem Becherglas gefüllt und (1 Tropfen/Sekunde) in das Becherglas getropft. Nach Zugabe von knapp der Hälfte der Natriumaluminatlösung (entsprechend der Stöchiometrie bei der in Beispiel 9 genannten Herstellung des "Neutralized Aluminum Salt D") war erst ein pH-Wert von 3,7 erreicht worden. Bei Zugabe des restlichen Natriumaluminats wurde das Reaktionsgemisch bei einem gemessenen pH-Wert von 5,7 fest.

Der Versuch wurde wederholt, wobei jedoch 60 g einer wie beschrieben hergestellten Natriumaluminatlösung auf einmal der Aluminiumsulfatlösung zugegeben wurden. Dadurch wurde ein pH-Wert von 3,7 erreicht. Danach wurde in Portionen von jeweils ungefähr 2 g weitere Natriumaluminatlösung zugegeben. Nach der Zugabe von insgesamt 85 g war ein pH von 4,3 erreicht und der Becherinhalt war fest.

Eine Suspension, die durch Versprühen auf Superabsorber aufgebracht werden könnte, wurde nicht erhalten.

Beispiel 12 (Vergleich)

In einem 150 mL-Becherglas wurden 36,83 g 26,8 Gew.-%ige wässrige Aluminiumsulfatlösung (enthaltend 28,9 mMol $Al_2(SO_4)_3$) vorgelegt und mit einem Rührfisch magnetisch gerührt. 12,24 g 50 Gew.-%ige Natronlauge (enthaltend 153 mMol NaOH) wurden in einen Tropftrichter über dem Becherglas gefüllt. Die Natronlauge wurde (1 Tropfen/Sekunde) in das Becherglas getropft. Nach Abschluss der Zugabe der Natronlauge wurden 16,68 g Wasser zugegeben und die entstehende Dispersion für 15 Minuten weiter gerührt. Abschließend wurden vereinzelte Klumpen durch einminütiges Rühren mit einem Ultra-Turrax® zerkleinert. Der anschließend gemessene pH-Wert betrug 6,65.

Beispiel 13 (Vergleich)

Beispiel 1 wurde wiederholt, wobei statt dem dort verwendeten röntgenamorphen Aluminiumhydroxidpulver das in Beispiel 12 hergestellte Aluminiumhydroxidsol aufgesprüht wurde. Die damit aufgegebene Aluminiumhydroxidmenge betrug 0,5 Gew.-% und die damit aufgegebene, zur Erzeugung einer sprühbaren Dispersion notwendige Wassermenge, betrug 5,3 Gew.-%, bezogen auf den Superabsorber. Die erhaltenen Messwerte sind in Tabelle 1 angegeben.

Auswertung

Beispiele 9, 10 und 11 zeigen, dass in den in Beispiel 9 genannten Schriften nicht oder jedenfalls nicht eindeutig Aluminiumhydroxidsol erzeugt und verwendet wurde. Beispiel 12 zeigt, dass größere Wassermengen als die in den in Beispiel 9 genannten Schriften genannten notwendig sind, um ein versprühbares Aluminiumhydroxidsol zu erhalten, wenn die Reaktanden auch annähernd in der theoretischen Stöchiometrie eingesetzt werden und sich bei einem pH-Wert im neutralen Bereich auch sicher Aluminiumhydroxidsol bilden kann. Beispiel 13 zeigt im Vergleich mit den nächstkommenden Beispielen 1 und 4, dass die Steigerung der GBP nicht oder jedenfalls weit weniger ausgeprägt erfolgt, wenn man dem Superabsorber Aluminiumhydroxidsol statt trockenem pulverförmigem Aluminiumhydroxid, wahlweise von Wasser gefolgt, zugibt.

Tabelle 1

| Beispiel | röntgenamorphes Al(OH)$_3$ [Gew.-%] | kristallines Al (OH)$_3$ [Gew.-%] | Nachbefeuchtung [Gew.-%] | CRC [g/g] | AUL0.9psi [g/g] | GBP [g/g] | VAUL $\tau$ (0.3psi) [s] |
|---|---|---|---|---|---|---|---|
| 1 | - | - | - | 29,1 | 23,8 | 17 | 161 |
|   | 0,5 | - | - | 28,2 | 20,7 | 70 | 155 |
| 2 | - | - | - | 30,5 | 23,0 | 17 | n.b. |
|   | 0,2 | - | 3,0 | 28,7 | 20,8 | 78 | 154 |
| 3 | - | - | - | 30,5 | 22,9 | 14 | n.b. |
|   | 0,35 | - | 3,0 | 28,8 | 20,2 | 86 | 154 |
| 4 | - | - | - | 28,6 | 23,2 | 18 | 173 |
|   | 0,5 | - | 3,0 | 27,1 | 20,3 | 94 | 174 |

(fortgesetzt)

| Beispiel | röntgenamorphes Al(OH)$_3$ [Gew.-%] | kristallines Al(OH)$_3$ [Gew.-%] | Nachbefeuchtung [Gew.-%] | CRC [g/g] | AUL0.9psi [g/g] | GBP [g/g] | VAUL $\tau$ (0.3psi) [s] |
|---|---|---|---|---|---|---|---|
| 5 | - | - | - | 29,6 | 22,8 | 14 | n.b. |
|   | 0,75 | - | 3,0 | 28,3 | 19,6 | 88 | 165 |
| 6 (Vergleich) | - | - | - | 28,9 | 23,5 | 19 | 162 |
|   | - | 0,5 | - | 27,9 | 22,4 | 23 | 166 |
| 7 | 0,5 | - | - | 30,5 | 18,2 | 112 | 177 |
| 8 (Vergleich) | - | 0,5 | - | 27,8 | 22,8 | 17 | 165 |
| 13 (Vergleich) | 0,5 (als Dispersion) | - | 5,3 (als Dispersion) | 28,8 | 23,2 | 26 | n.b. |

**Patentansprüche**

**1.** Verfahren zur Herstellung eines Superabsorbers, umfassend

Polymerisation einer wässrigen Monomerlösung, die

a) mindestens ein ethylenisch ungesättigtes, säuregruppentragendes Monomer, das wahlweise zumindest teilweise als Salz vorliegt,
b) mindestens einen Vernetzer,
c) mindestens einen Initiator,
d) wahlweise ein oder mehrere mit den unter a) genannten Monomeren copolymerisierbare ethylenisch ungesättigte Monomere, und
e) wahlweise ein oder mehrere wasserlösliche Polymere enthält;

Trocknung des erhaltenen Polymerisats,
wahlweise Mahlung des getrockneten Polymerisats und Siebung des gemahlenen Polymerisats, und
wahlweise Oberflächennachvernetzung des getrockneten und gegebenenfalls gemahlenen und gesiebten Polymerisats,
das **dadurch gekennzeichnet ist, dass** man nach Trocknung, Mahlung oder Siebung und sofern eine Oberflächennachvernetzung durchgeführt wird vor, während oder nach dieser Oberflächennachvernetzung röntgenamorphes Aluminiumhydroxidpulver zugibt.

**2.** Verfahren nach Anspruch 1, **dadurch gekennzeichnet, dass** man 0,01 bis 2 Gew.-%, bezogen auf die Polymerisatmenge vor Zugabe, röntgenamorphes Aluminiumhydroxidpulver zugibt.

**3.** Verfahren nach Anspruch 2, **dadurch gekennzeichnet, dass** man 0,2 bis 1 Gew.-%, bezogen auf die Polymerisatmenge vor Zugabe, röntgenamorphes Aluminiumhydroxidpulver zugibt.

**4.** Verfahren nach einem der Ansprüche 1 bis 3, **dadurch gekennzeichnet, dass** man das getrocknete und gegebenenfalls gemahlene und gesiebte Polymerisat mit Nachvernetzern, die kovalente Bindungen mit polaren Gruppen an der Oberfläche der Superabsorberpartikel bilden, an seiner Oberfläche nachvernetzt.

**5.** Verfahren nach einem der Ansprüche 1 bis 4, **dadurch gekennzeichnet, dass** man dem Superabsorber nach der Zugabe von röntgenamorphem Aluminiumhydroxidpulver 0,1 bis 10 Gew.-%, bezogen auf die Polymerisatmenge vor Zugabe des röntgenamorphen Aluminiumhydroxidpulvers, Wasser zusetzt.

**6.** Superabsorber, erhältlich nach dem Verfahren eines der Ansprüche 1 bis 5.

7. Artikel zur Absorption von Flüssigkeiten, enthaltend den in Anspruch 6 definierten Superabsorber.

8. Verfahren zur Herstellung von Artikeln zur Absorption von Flüssigkeit, **dadurch gekennzeichnet, dass** man bei der Herstellung der Artikel ihnen den in Anspruch 6 definierten Superabsorber zusetzt.

**Claims**

1. A process for producing a superabsorbent, comprising

   polymerizing an aqueous monomer solution comprising

      a) at least one ethylenically unsaturated monomer which bears acid groups and is optionally at least partly in salt form,
      b) at least one crosslinker,
      c) at least one initiator,
      d) optionally one or more ethylenically unsaturated monomers copolymerizable with the monomers mentioned under a), and
      e) optionally one or more water-soluble polymers;

   drying the resulting polymer,
   optionally grinding the dried polymer and sieving the ground polymer, and
   optionally surface postcrosslinking the dried and optionally ground and sieved polymer,
   wherein, after drying, grinding or sieving, and, if surface postcrosslinking is conducted, prior to, during or after this surface postcrosslinking, x-ray-amorphous aluminum hydroxide powder is added.

2. The process according to claim 1, wherein 0.01% to 2% by weight, based on the amount of polymer prior to addition, of x-ray-amorphous aluminum hydroxide is added.

3. The process according to claim 2, wherein 0.2% to 1% by weight, based on the amount of polymer prior to addition, of x-ray-amorphous aluminum hydroxide is added.

4. The process according to any of claims 1 to 3, wherein the dried and optionally ground and sieved polymer is surface postcrosslinked with postcrosslinkers that form covalent bonds with polar groups at the surface of the superabsorbent particles.

5. The process according to any of claims 1 to 4, wherein, after the addition of x-ray-amorphous aluminum hydroxide powder, 0.1% to 10% by weight, based on the amount of polymer prior to addition of the x-ray-amorphous aluminum hydroxide powder, of water is added to the superabsorbent.

6. A superabsorbent obtainable by the process of any of claims 1 to 5.

7. An article for absorption of fluids, comprising the superabsorbent defined in claim 6.

8. A process for producing articles for absorption of fluid, wherein the production of the articles involves addition of the superabsorbent defined in claim 6.

**Revendications**

1. Procédé pour la préparation d'un superabsorbant, comprenant

   la polymérisation d'une solution aqueuse de monomères qui contient

      a) au moins un monomère éthyléniquement insaturé, portant des groupes de type acide qui est présent éventuellement au moins partiellement en tant que sel,
      b) au moins un agent de réticulation,
      c) au moins un initiateur,

d) éventuellement un ou plusieurs monomères éthyléniquement insaturés copolymérisables avec les monomères mentionnés en a), et

e) éventuellement un ou plusieurs polymères solubles dans l'eau ;

le séchage du polymérisat obtenu,

éventuellement le broyage du polymérisat séché et le tamisage du polymérisat broyé, et

éventuellement la post-réticulation en surface du polymérisat séché et éventuellement broyé et tamisé, qui est **caractérisé en ce que**, après le séchage, le broyage ou le tamisage et pour autant qu'une post-réticulation en surface est réalisée, on ajoute de la poudre d'hydroxyde d'aluminium amorphe aux rayons X avant, pendant ou après cette post-réticulation en surface.

2. Procédé selon la revendication 1, **caractérisé en ce qu'**on ajoute 0,01 à 2 % en poids de poudre d'hydroxyde d'aluminium amorphe aux rayons X, par rapport à la quantité de polymérisat avant l'ajout.

3. Procédé selon la revendication 2, **caractérisé en ce qu'**on ajoute 0,2 à 1 % en poids de poudre d'hydroxyde d'aluminium amorphe aux rayons X, par rapport à la quantité de polymérisat avant l'ajout.

4. Procédé selon l'une quelconque des revendications 1 à 3, **caractérisé en ce qu'**on post-réticule le polymérisat séché et éventuellement broyé et tamisé, au niveau de sa surface, avec des agents de post-réticulation qui forment des liaisons covalentes avec des groupes polaires au niveau de la surface des particules de superabsorbant.

5. Procédé selon l'une quelconque des revendications 1 à 4, **caractérisé en ce qu'**on ajoute 0,1 à 10 % en poids d'eau au superabsorbant après l'ajout de poudre d'hydroxyde d'aluminium amorphe aux rayons X, par rapport à la quantité de polymérisat avant l'ajout de la poudre d'hydroxyde d'aluminium amorphe aux rayons X.

6. Superabsorbant, pouvant être obtenu par le procédé selon l'une des revendications 1 à 5.

7. Article pour l'absorption de liquides, contenant le superabsorbant défini dans la revendication 6.

8. Procédé pour la préparation d'articles pour l'absorption de liquide, **caractérisé en ce que**, lors de la fabrication des articles, on ajoute à ceux-ci le superabsorbant défini dans la revendication 6.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7795345 B2
- US 3932322 A
- WO 2013076031 A1
- WO 0168156 A1
- WO 200774108 A1
- WO 2007121941 A2
- WO 9955767 A1
- WO 9848857 A1
- WO 0174913 A1
- US 6620889 B1
- WO 2006111402 A2
- WO 2005108472 A1
- WO 2004113452 A1
- WO 2013156281 A1
- WO 2010108875 A1
- WO 2012045705 A1
- WO 2013156330 A1
- WO 2009080611 A2
- EP 233067 A2
- US 5145906 A
- JP 9124879 A
- EP 780424 A1
- US 5684106 A
- JP 3121934 B
- WO 03049778 A1
- WO 2012143215 A1
- WO 201372311 A1
- WO 2014167036 A1
- WO 2014167040 A
- WO 2014168858 A1
- US 20160235882 A1
- WO 2002055469 A1
- WO 2003078378 A1
- WO 2004035514 A1
- EP 530438 A1
- EP 547847 A1
- EP 559476 A1
- EP 632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 200232962 A2
- WO 200138402 A1
- EP 955086 A2
- DE 3825366 A1
- US 6241928 B
- EP 445619 A2
- DE 19846413 A1
- EP 457660 A1
- EP 348180 A1
- EP 816383 A1
- WO 9640427 A1
- US 4020256 A
- US 20020193546 A1
- DE 3519013 A1
- DE 102005044035 A1
- WO 2007093531 A1
- WO 2008086976 A1
- WO 2009027356 A1
- WO 0294328 A2
- WO 0294329 A1
- EP 0534228 A1
- EP 1191051 A2
- US 20030181115 A
- US 20040019342 A
- EP 0377199 A2
- EP 0445641 A1
- US 5026806 A
- EP 0655465 A1
- EP 0377191 A2
- US 20070135785 A1
- US 20140306156 A1
- US 20150299404 A1
- EP 0640330 A1
- US 20050256757 A1
- WO 2014167040 A1

**In der Beschreibung aufgeführte Nicht-Patentliteratur**

- **FREDRIC L ; BUCHHOLZ ; ANDREW T. GRAHAM.** Modern Superabsorbent Polymer Technology. J. Wiley & Sons, Wiley-VCH, 1997

- Sauerstoffverbindungen des Aluminiums. **HOLLEMAN ; WIBERG.** Lehrbuch der Anorganischen Chemie. Walter de Gruyter & Co, 2016, 103
- Aluminum Oxide. **STICHWORT.** Ullmann's Encyclopedia of Industrial Chemistry. Wiley-VCH Verlag Gmbh & Co. KGaA, 2012
- **T. ISOBE et al.** *J. Colloid and Interface Science,* 2003, vol. 261, 320-324
- Structure Analysis by Diffraction. **STICHWORT.** Ullmann's Encyclopedia of Industrial Chemistry. Wiley-VCH Verlag Gmbh & Co. KGaA, 2012